(19) Europäisches Patentamt
European Patent Office
Office européen des brevets

(11) EP 1 284 265 A1

(12) **EUROPEAN PATENT APPLICATION**
published in accordance with Art. 158(3) EPC

(43) Date of publication:
**19.02.2003 Bulletin 2003/08**

(21) Application number: **01912488.2**

(22) Date of filing: **19.03.2001**

(51) Int Cl.[7]: **C07D 333/60**, A61K 31/381, A61K 31/5377, A61P 43/00, A61P 3/06, A61P 9/10

(86) International application number:
**PCT/JP01/02170**

(87) International publication number:
**WO 01/070723 (27.09.2001 Gazette 2001/39)**

(84) Designated Contracting States:
**AT BE CH CY DE DK ES FI FR GB GR IE IT LI LU MC NL PT SE TR**

(30) Priority: **22.03.2000 JP 2000079194**

(71) Applicant: **Mitsui Chemicals, Inc.**
**Tokyo 100-6070 (JP)**

(72) Inventors:
- **TSUNODA, Hidetoshi, c/o Mitsui Chemicals, Inc.**
  **Mobara-shi, Chiba 297-0017 (JP)**
- **FUKAZAWA, Nobuyuki,**
  **c/o Mitsui Chemicals, Inc.**
  **Tokyo 100-6070 (JP)**
- **CHIBA, Kyoko, c/o Mitsui Chemicals, Inc.**
  **Mobara-shi, Chiba 297-0017 (JP)**
- **NAKAO, Toshifumi, c/o Mitsui Chemicals, Inc.**
  **Mobara-shi, Chiba 297-0017 (JP)**
- **ASADA, Noriaki, c/o Mitsui Chemicals, Inc.**
  **Mobara-shi, Chiba 297-0017 (JP)**
- **TAKEBAYASHI, Nozomi,**
  **c/o Mitsui Chemicals, Inc.**
  **Mobara-shi, Chiba 297-0017 (JP)**
- **KIBAYASHI, Kenji**
  **Suita-shi, Osaka 565-0836 (JP)**
- **MIGITA, Hideyuki**
  **Mobara-shi, Chiba 297-0017 (JP)**
- **MORIKAWA, Maki**
  **Mobara-shi, Chiba 297-0012 (JP)**

(74) Representative: **Stuart, Ian Alexander et al**
**MEWBURN ELLIS**
**York House**
**23 Kingsway**
**London WC2B 6HP (GB)**

(54) **BENZOTHIOPHENE DERIVATIVES AND MEDICINAL USE THEREOF**

(57) A benzothiophene derivative represented by formula (1):

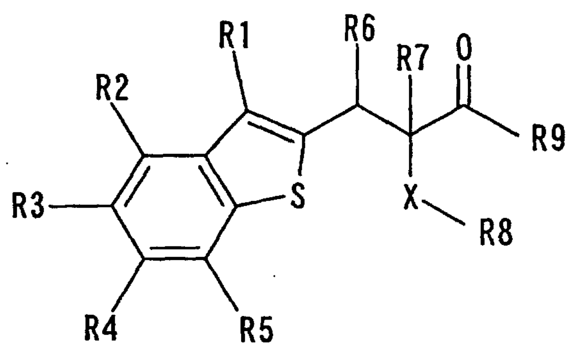

(1)

can activate a peroxisome proliferator-activated receptor (PPAR) α or γ which is an intranuclear transcription factor. The compound may be used as an active ingredient to give a drug for preventing or treating a variety of diseases involving the receptor.

EP 1 284 265 A1

## Description

Technical Field

This invention relates to a novel benzothiophene derivative effective for prevention and treatment of a variety of diseases demonstrating pharmacological effects by α- or γ-activation of a peroxisome proliferator-activated receptor (hereinafter, referred to as "PPAR") which is an intracellular transcription factor to various cells in an organism. "A variety of diseases" as used herein include in particular hypoglycemia and hypolipidemia in diabetes, complications in diabetes, hyperlipidemia, arteriosclerosis and various thromboses as well as a wide variety of inflammatory diseases such as rheumatoid arthritis, hypertrophic arthritis, asthma, bronchitis, allergic diseases, inflammatory diseases of an internal organ, ulcerative colitis, Crohn's disease, sepsis, septic shock, Kepler tuberculosis, multiple sclerosis, ischemic vascular disorders including DIC, cerebral malaria, hepatitis, cancers, autoimmune disease and cachexia associated with a cancer or viral disease including AIDS.

Background Art

Diabetics have been increased due to current change in life style. In Japan, there are about 7 million patients. It is believed that there are 13 million or more patients including those around a borderline (a recent report by the study group for diabetes in the Ministry of Health and Welfare has described that about 10 % of the population over 40 years has diabetes in Japan; Progress in Diabetes '96 (Vol. 30); Shindan-to-Chiryo Company, Tokyo, p.25, 1996). In global level, this tendency can be also observed. Thus, it is socially urgent to deal with the problem in the light of a coming aging society.

A pathology of diabetes may be said to be a persistent hyperglycemia due to absolute and relative insufficiency in insulin. The persistent hyperglycemia causes a variety of chronic complications such as nephropathy, retinopathy and neuropathy, which may in turn make the pathology more complicated and severe (Diabetes Mellitus Metabolism, Vol. 36, Suppl. 1, p.22, 1987). For controlling such conditions, it is important to develop a drug for improving glucose metabolism and preventing a persistent hyperglycemia condition. Diabetes can be pathologically divided into two types: insulin dependent (Type I) and non-insulin independent (Type II) types. Most of diabetes in Japan belongs to Type II, i.e., non-insulin dependent diabetes. It is known that Type II diabetes is caused by insulin resistance or insulin secretion insufficiency, and thus therapeutic drugs have been investigated from these two approaches.

Insulin secretion insufficiency has been treated by insulin therapy as well as by using well-known and common sulfonyl ureas (SU) such as Tolbutamide, Acetohexamide, Glibenclamide (Oral Hypoglycemic Agents, N. Engl. J. Meg., Vol. 321, p.1231, 1989). SU drugs exhibit potent hypoglycemic effect, but may cause a serious side effect, hypoglycemia (Diabetic Med., Vol. 5, p.315, 1988), and thus cannot be conveniently used. Prolonged use of an SU drug may cause secondary problems such as adiposis acceleration (Curr. Opin. Nephrol. Hypertens., Vol. 1, p. 291, 1992).

Biguanides such as Fenformin and Metformin have been used for insulin resistance. These biguanides are believed to be clinically impractical due to drawbacks such as insufficient hypoglycemic effect and tendency to inducing serious lactate-acidosis (Diabetic Med., Vol. 5, p.315, 1988; Practice, Vol. 13, p.331, 1996).

For solving these drawbacks, several drugs having a thiazolidinedione structure have been clinically used as a novel insulin resistance improving agent (drugs such as Troglitazone and Pioglitazone ; JP-As 55-22626, 60-51189, 6-157522 and so on). In addition to these thiazolidinedione drugs, there have been also developed isoxazole-ring containing compounds (WO 95/18125), phenylpropionic acid derivatives (WO 93/21166, WO 96/-4260, JP-A 11-158144), malonic acid derivatives (JP-A 9-323982), and tyrosine derivatives (JP-A 8-325263). These drugs, however, also exhibit insufficient effects and suffer from problems in practical use such as hepatotoxicity and adverse effects to a circulatory system (Lancet, Vol. 350, p.1748, 1997).

Besides, of course, prolonged hyperglycemia, contribution of free fatty acids and neutral lipid in blood has been recently emphasized as a factor triggering insulin resistance (Prostaglandins Leukotriens Essent. Fatty Acid, Vol. 53, p.385, 1995). It has been gradually acknowledged that a drug for effectively improving insulin resistance must have not only hypoglycemic effect but also hypolipidemic effect.

To date, it has been found that PPAR have several subtypes including PPARα, PPARβ(δ) and PPARγ (Latruffe N. and Vamecq J., Biochimie, Vol. 79, p.81, 1997). It has been recently believed that a PPARα activator exhibits hypolipidemic effect primarily by promoting lipid metabolism. For example, clinically-applied fibrate hyperlipidemia drugs such as chlofibrate and bezafibrate exhibit weak PPARα activating effect, which is believed to be one of the mechanisms for their pharmacological effects. Furthermore, it is believed that hypoglycemic effect of the above insulin resistance improving agents (thiazolidine drugs) may be partially derived from PPARγ activation. It has been thus demonstrated that PPARs play an important role in glucose metabolism or lipid metabolism in a living body.

In addition to known involvement in lipid metabolism and glucose metabolism, both PPARα and PPARγ have been found to be involved in a wide variety of inflammatory cells (Igaku no Ayumi, Vol. 190, No.10, p.928, 1999),

implying their application to novel anti-inflammatory agents based on a new mechanism.

Thus, PPARα or γ activators are promising as an agent for preventing or treating the above various diseases, but have drawbacks insufficient efficacy and limitation in patients to which they are effective, because known agents are effective to only one PPAR subtype (α or γ) or insufficiently activate them. They have many problems as a medical drug in terms of toxicity, pharmacokinetics and so on. There have been, therefore, needed to develop more effective agents for preventing or treating the above diseases, which can be applied to a wider range of patients.

There have been many reports for compounds having a benzothiophene structure, some of which have been clinically used as a medical drug. Examples include Raloxifene hydrochloride as an antiestrogen (Eli Lilly & Co.); Sertaconazole nitrate (Ferrer Co.) as an antibacterial agent; and Zileuton (Abbott) as an anti-inflammatory agent. We have also reported certain benzothiophene derivatives as a cell adhesion inhibitor in JP-A 10-175970. There have been no descriptions that these compounds have PPARα or γ activating effect and that on the basis of such an effect, they can ameliorate hypoglycemia and hypolipidemia in diabetes, complications in diabetes, hyperlipidemia, arteriosclerosis and various thromboses as well as a wide variety of inflammatory diseases such as rheumatoid arthritis, hypertrophic arthritis, asthma, bronchitis, allergic diseases, inflammatory diseases of an internal organ, ulcerative colitis, Crohn's disease, sepsis, septic shock, Kepler tuberculosis, multiple sclerosis, ischemic vascular disorders including DIC, cerebral malaria, hepatitis, cancers, autoimmune disease and cachexia associated with a cancer or viral disease including AIDS.

Disclosure of the Invention

Thus, an objective of this invention is to provide a novel benzothiophene derivative exhibiting PPAR α or γ activating effect and particularly useful as an agent for preventing or treating hypoglycemia and hypolipidemia in diabetes and its complications, hyperlipidemia, arteriosclerosis and various thromboses, as well as a wide variety of inflammatory diseases such as rheumatoid arthritis, hypertrophic arthritis, asthma, bronchitis, allergic diseases, inflammatory diseases of an internal organ, ulcerative colitis, Crohn's disease, sepsis, septic shock, Kepler tuberculosis, multiple sclerosis, ischemic vascular disorders including DIC, cerebral malaria, hepatitis, cancers, autoimmune disease and cachexia associated with a cancer or viral disease including AIDS.

The present inventors have intensely attempted to provide compounds exhibiting PAPRα or γ activating effect and hypoglycemic and hypolipidemic effects in a cell level and in a variety of an animal level, and have finally found that novel benzothiophene derivatives according to this invention exhibit potent PAPRα or γ activating effect as well as potent hypoglycemic and hypolipidemic effects. We have further found that these compounds are nontoxic and very useful as an orally-absorbable drug, achieving this invention.

This invention provides a benzothiophene derivative represented by formula (1):

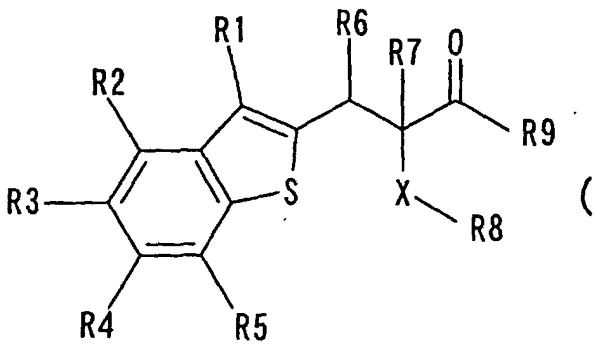

(1)

wherein $R^1$, $R^2$, $R^3$, $R^4$ and $R^5$ independently represent hydrogen, halogen, hydroxy, lower alkyl having 1 to 4 carbon atoms, lower alkyloxy having 1 to 4 carbon atoms, nitro, carboxyl, optionally substituted carbamoyl, lower alkoxycarbonyl having 1 to 4 carbon atoms, optionally substituted phenoxy, thiol, lower alkylthiol having 1 to 4 carbon atoms, optionally substituted phenylthio, amino optionally substituted with lower alkyl having 1 to 4 carbon atoms, amino optionally substituted with lower alkylcarbonyl having 1 to 4 carbon atoms or amino substituted with optionally substituted benzoyl; $R^6$ represents hydrogen or lower alkyl having 1 to 4 carbon atoms; $R^7$ represents hydrogen, lower alkyl having 1 to 4 carbon atoms or lower alkyloxy having 1 to 4 carbon atoms; or $R^6$ and $R^7$ may be directly bound together to form a carbon-carbon double bond; $R^8$ represents hydrogen, lower alkyl having 1 to 4 carbon atoms, optionally substituted phenyl, lower alkyloxy having 1 to 4 carbon atoms, amino optionally substituted with lower alkyl having 1 to 4 carbon atoms or optionally substituted phenylamino; $R^9$ represents hydroxy, lower alkyloxy having 1 to 4 carbon atoms, optionally substituted phenyloxy, amino optionally substituted with lower alkyl having 1 to 4 carbon atoms or optionally substituted phenylamino; and X represents oxygen, sulfur or carbonyl.

Pharmaceutically acceptable salts of the above benzothiophene derivatives are also included in this invention.

Preferred embodiments of the benzothiophene derivatives according to this invention include;

1. benzothiophene derivatives represented by formula (2):

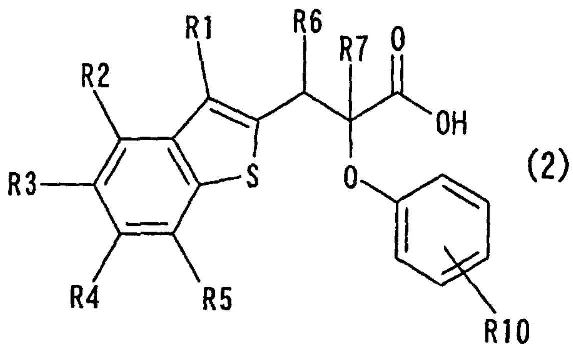

(2)

wherein $R^1$, $R^2$, $R^3$, $R^4$, $R^5$, $R^6$ and $R^7$ are as defined in formula (1); $R^{10}$ represents hydrogen, hydroxy, lower alkyl having 1 to 4 carbon atoms, lower alkoxy having 1 to 4 carbon atoms, nitro, halogen, carboxyl, lower alkoxycarbonyl having 1 to 4 carbon atoms, optionally substituted phenyl; optionally substituted amino, optionally substituted carbamoyl or optionally substituted amidino; and

2. compounds represented by formula (3):

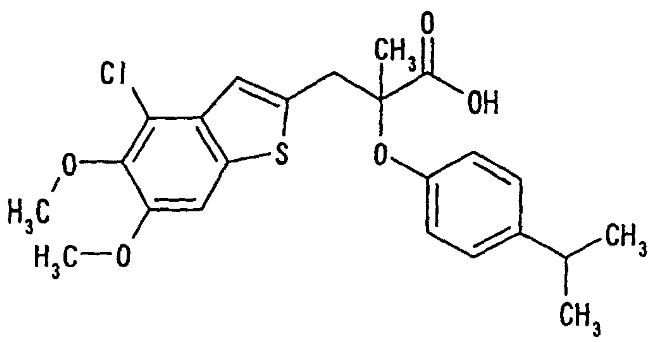

(3)

Benzothiophene derivatives according to this invention and their pharmaceutically acceptable salts are useful as an active ingredient for a medical drug. For example, a benzothiophene derivative according to this invention or its pharmaceutically acceptable salt may be used to prepare an agonist for a peroxisome proliferator-activated receptor (PPAR) $\alpha$ or $\gamma$ which is an intranuclear transcription factor, a drug for preventing or treating diabetes, a drug for preventing or treating hyperlipidemia and a drug for preventing or treating arteriosclerosis.

Best Mode for Carrying Out the Invention

This invention will be described in detail.

In terms of the substituents in formula (1), lower alkyl groups having 1 to 4 carbon atoms include methyl, ethyl, propyl, isopropyl and butyl groups. Lower alkyloxy groups having 1 to 4 carbon atoms include methoxy, ethoxy, propoxy and butoxy groups. Alkylcarbonyl groups having 1 to 4 carbon atoms include acetyl, propionyl and butanoyl groups. Halogen atoms include fluorine, chlorine, bromine and iodine atoms. A pharmaceutically acceptable salt may be any nontoxic salt formed with the compound of this invention. Examples of salts with an acidic functional group include inorganic base salts such as sodium, potassium, calcium and magnesium salts; and organic base salts such as ammonium, trimethylamine, pyridine, picoline, dicyclohexylamine, lysine and arginine salts. Examples of salts with a basic functional group include inorganic acid salts such as hydrochloride, sulfate and nitrate; and organic acid salts such as

acetate, fumarate, malonate, tartrate, oxalate and maleate.

Processes for preparing the compounds of this invention will be described.

Synthetic method 1

A saturated benzothiophene derivative represented by formula (5a) or (5b) which is a compound of this invention or an important intermediate for preparing the compound may be prepared by any of the following processes.

Synthetic method 1-1

A carboxylic acid derivative represented by formula (3a) is reacted with a 2-halomethylbenzothiophene derivative represented by formula (4) under a basic condition to provide the saturated benzothiophene derivative represented by formula (5a);

Synthetic method 1-2

A carboxylic acid derivative represented by formula (3a) is reacted with a benzothiophene-2-aldehyde or ketone derivative represented by formula (6) under a basic condition to produce an alcohol derivative represented by formula (7a), which then undergoes dehydroxylation to provide the saturated benzothiophene derivative represented by formula (5a);

Synthetic method 1-3

A carboxylic acid derivative represented by formula (3b) is condensed by dehydration with a benzothiophene-2-aldehyde or ketone derivative represented by formula (6) under an acidic or basic condition to produce an unsaturated benzothiophene derivative represented by formula (8), which is then reduced by, for example, catalytic hydrogenation to provide a saturated benzothiophene represented by formula (5b).

Examples of these processes are illustrated in the following Reaction Schemes (1) to (3):

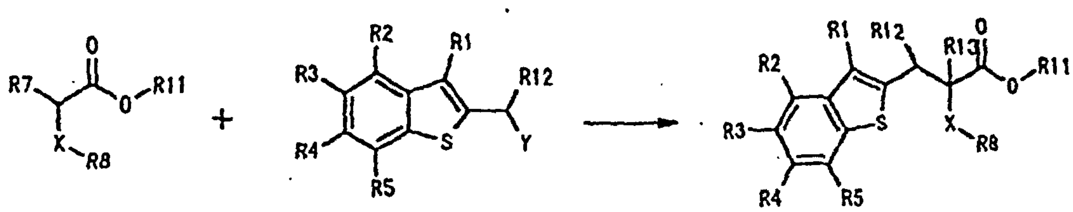

(3a) (4) (5a)

Reaction Scheme (1)

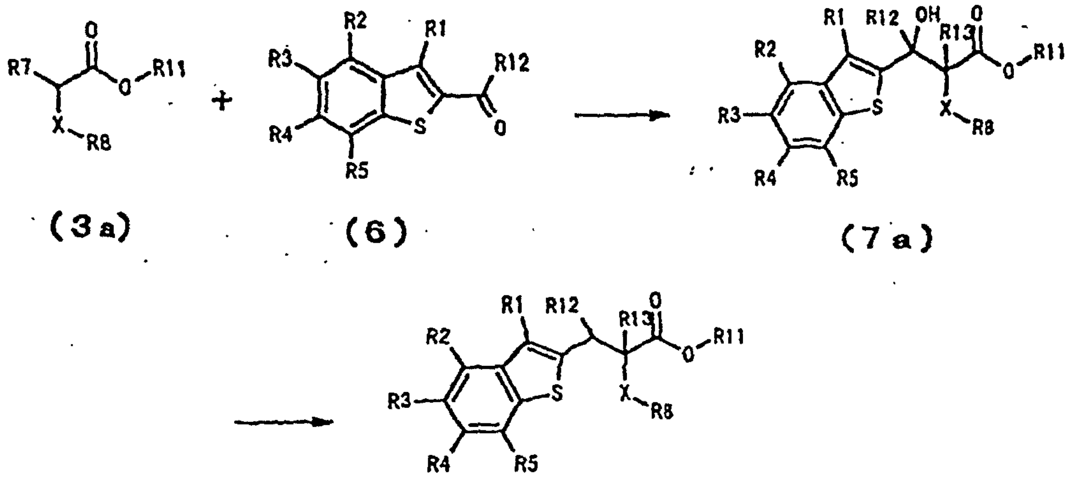

(3a) (6) (7a)

(5a) Reaction Scheme (2)

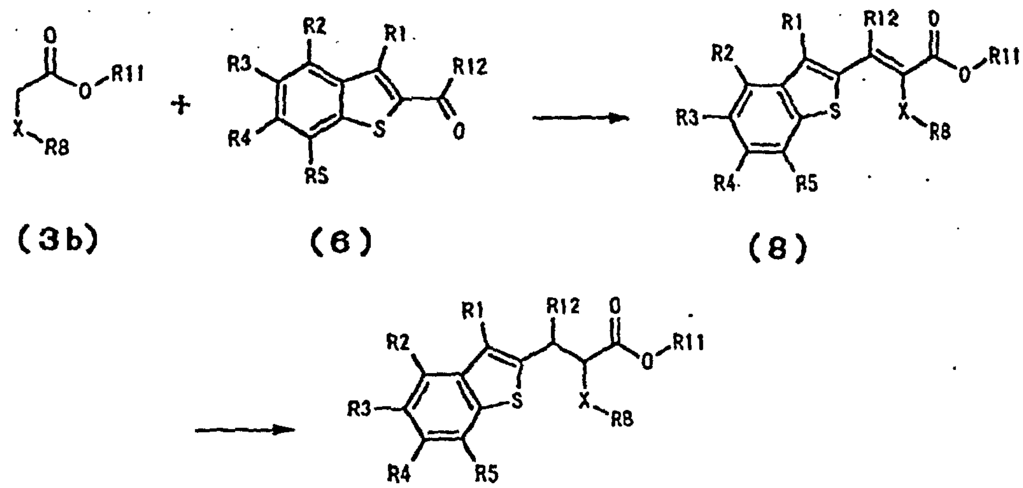

(3b) (6) (8)

(5b) Reaction Scheme (3)

wherein $R^1$, $R^2$, $R^3$, $R^4$, $R^5$, $R^7$, $R^8$ and X are as defined above; $R^{11}$ represents lower alkyl with 1 to 4 carbon atoms or optionally substituted phenyloxy; $R^{12}$ represents hydrogen or lower alkyl having 1 to 4 carbon atoms; $R^{13}$ represents hydrogen, lower alkyl having 1 to 4 carbon atoms or lower alkyloxy having 1 to 4 carbon atoms; and Y represents halogen.

The reactions involved in the above reaction schemes will be individually described.

i. Reaction Scheme (1)

The compounds represented by formulas (3a), (3b), (4) and (6) as starting materials can be prepared by known procedures or similar methods (for preparation of the compounds represented by formulas (3a) and (3b), see, for example, J. Med. Chem., Vol. 39, p.4783, 1996; for preparation of the compounds represented by formulas (4) and (5), see, for example, JP-A 60-209577, JP-A 2-085281, WO 95-15323, J. Med. Chem. Vol. 35, p.958, 1992).

Examples of a base which can be used for preparing the compound represented by formula (5a) include, but not limited to, alkali or alkaline earth metals such as sodium and potassium; alkali or alkaline earth metal hydrides such as sodium hydride and potassium hydride; metal alkoxides such as sodium methoxide, sodium ethoxide and potassium tert-butoxide; inorganic bases such as sodium hydroxide, potassium hydroxide, sodium carbonate and cesium carbonate; organic bases such as pyridine, triethylamine, 1,8-diazabicyclo-7-undecene (hereinafter, referred to as "DBU"); and alkali metal amides such as lithium diisopropylamide (hereinafter, referred to as "LDA"), lithium isopropylcyclohexylamide (hereinafter, referred to as "LICA") and lithium hexamethyldisilazide (hereinafter, referred to as "LiHMDS").

Examples of a solvent which can be used include, but not limited to, protic solvents such as water, methanol and ethanol; and aprotic solvents such as pyridine, triethylamine, tetrahydrofuran (hereinafter, referred to as "THF"), dimethylformamide (hereinafter, referred to as "DMF"), diethyl ether, dimethylsulfoxide (hereinafter, referred to as "DMSO"), dichloromethane, chloroform and toluene. The reaction may be conducted at a temperature in the range of -100 °C to the boiling point of a solvent used, preferably in the range of -100 °C to room temperature.

ii. Reaction Scheme 2

There are no restrictions to bases and solvents which can be used for preparing the compounds represented by formula (7a) and those as described for the compounds represented by formula (5a) can be used. The reaction can be conducted at a temperature in the range of -100 °C to the boiling point of a solvent used, preferably in the range of -100 °C to room temperature.

Reduction of the hydroxy group in the compound represented by formula (7a) can be conducted either by a direct reduction or by forming a leaving group before reduction. Examples of direct reduction include using an alkylsilane such as triethylsilane in the presence of an acid catalyst and using any of various hydrogenation metal catalyst under the hydrogen atmosphere. Examples of the acid catalyst include inorganic proton acids such as hydrochloric acid, sulfuric acid and nitric acid; organic proton acids such as trifluoroacetic acid, trifluoromethanesulfonic acid and p-toluenesulfonic acid; and Lewis acids such as boron trifluoride. There are no restrictions to a solvent used in this reaction, and examples include protic solvents such as trifluoroacetic acid and acetic acid; and aprotic solvents such as tetrahydrofuran, dichloromethane, chloroform and diethyl ether. A reaction temperature may be in the range of -20 °C to the boiling point of a solvent used. Alternatively, a variety of hydrogenating metal catalysts may be used under a hydrogen atmosphere, including palladium-carbon, platinum oxide and Raney nickel. A solvent used may be selected without limitations; for example, protic solvents such as water, methanol and ethanol, and aprotic solvents such as ethyl acetate, THF and DMF.

As the method in which the reduction is conducted after the introduction of a leaving group, a method using any of various hydrogenating catalysts such as palladium-carbon, platinum oxide and Raney nickel under a hydrogen atmosphere, as described above, either after a halide is formed by a commonly used halogenating agent for a hydroxy group, or after a sulfonate is formed by a commonly used sulfonating agent for a hydroxy group. Alternatively, reduction may be conducted using a hydride such as lithium aluminum hydride and sodium borohydride.

iii. Reaction Scheme 3

There are no restrictions to a base or acid used for preparing the compound represented by formula (8). Examples of a base include alkali or alkaline earth metals such as sodium and potassium; alkali or alkaline earth metal hydrides such as sodium hydride and potassium hydride; metal alkoxides such as sodium methoxide, sodium ethoxide and potassium tert-butoxide; inorganic bases such as sodium hydroxide, potassium hydroxide, sodium carbonate and cesium carbonate; organic bases such as pyridine and DBU; and alkali metal amides such as LDA, LICA and LiHMDS. Examples of an acid include inorganic proton acids such as hydrochloric acid, sulfuric acid and nitric acid; organic proton acids such as acetic acid, methanesulfonic acid, p-toluenesulfonic acid, trifluoroacetic acid and trifluoromethanesulfonic acid ; and Lewis acids such as boron trifluoride. Examples of a solvent which may be used include, but not limited to, protic solvents such as water, methanol and ethanol; and aprotic solvents such as pyridine, triethylamine, THF, DMF, diethyl ether, DMSO, dichloromethane. chloroform and toluene. The reaction may be conducted at a temperature in the range of -20 °C to the boiling point of a solvent used.

Saturation of the unsaturated bond in the compound represented by formula (8) can be conducted as described above using any of various hydrogenation metal catalysts such as palladium-carbon, platinum oxide and Raney nickel under a hydrogen atmosphere. Alternatively, the process may be conducted using magnesium in a protic solvent such as an alcohol including methanol and ethanol, to effectively provide the compound represented by formula (5b). The reaction may be conducted at a temperature in the range of -20 °C to the boiling point of a solvent used.

Synthetic method 2

The unsaturated benzothiophene represented by formula (8) which is a compound of this invention or an important intermediate for preparing the compound may be prepared by any of the following processes.

Synthetic method 2-1

A carboxylic acid derivative represented by formula (3b) is reacted with a benzothiophene-2-aldehyde or

ketone derivative represented by formula (6) under a basic condition to produce an alcohol represented by formula (7b), which is then dehydrated to provide an unsaturated benzothiophene derivative represented by formula (8).

Synthetic method 2-2

As illustrated above, a carboxylic acid derivative represented by formula (3b) is condensed by dehydration with a benzothiphene-2-aldehyde derivative under a basic or acidic condition to provide an unsaturated benzothiophene derivative represented by formula (8).

Examples of these processes are illustrated by Reaction Schemes (4) and (5).

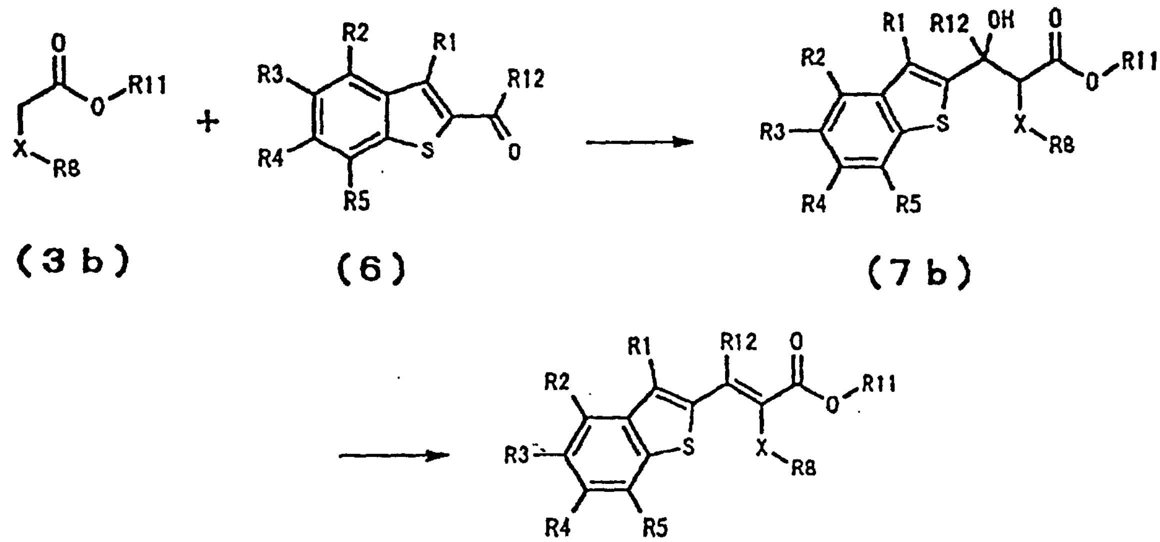

(3 b) (6) (7 b)

(8)

Reaction Scheme (4)

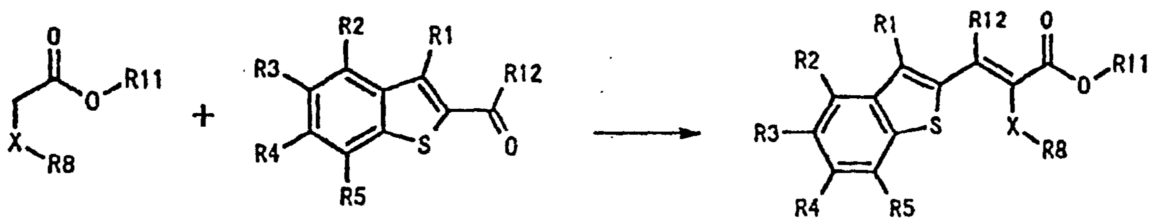

(3 b) (6) (8)

Reaction Scheme (5)

wherein $R^1$, $R^2$, $R^3$, $R^4$, $R^5$, $R^8$, $R^{11}$, $R^{12}$ and X are as defined above.

The reactions according to these reaction schemes will be individually described.

i. Reaction Scheme (4)

There are no restrictions to bases which can be used for preparing the compounds represented by formula (7b) and those as described for preparing the compound represented by formula (7a) in Reaction Scheme (2) can be used. Synthesis of the compound represented by formula (8) from the compound represented by formula (7b) uses dehydration reaction for which a method by direct dehydration using an acid and a method by forming an unsaturated bond by treatment with a base, once after formation of a derivative having a leaving group.

Examples of acids which can be used in the direct dehydration include, but not limited to, inorganic proton

acids such as hydrochloric acid, sulfonic acid and nitric acid; organic proton acids such as acetic acid, methanesulfonic acid, p-toluenesulfonic acid, trifluoroacetic acid and trifluoromethanesulfonic acid; and Lewis acids such as boron trifluoride. Examples of solvents which can be used include, but not limited to, protic solvents such as water, methanol and ethanol; and aprotic solvents such as pyridine, triethylamine, THF, DMF, diethyl ether, DMSO, dichloromethane, chloroform and toluene. The reaction can be conducted at a temperature in the range of -20 °C to the boiling point of a solvent used.

The process of first forming a leaving group may be generally conducted by, but not limited to, derivatizing a hydroxy group into a leaving group; for example, the process described for preparing the compound represented by formula (5a) from the compound represented by formula (7a).

Examples of bases which can be used in the reaction for forming an unsaturated bond include, but not limited to, alkali or alkaline earth metals such as sodium and potassium; alkali or alkaline earth metal hydrides such as sodium hydride and potassium hydride; metal alkoxides such as sodium methoxide, sodium ethoxide and potassium tert-butoxide; inorganic bases such as sodium hydroxide, potassium hydroxide, sodium carbonate and cesium carbonate; organic bases such as pyridine and DBU; and alkali metal amides such as LDA, LICA and LiHMDS. Examples of solvents which can be used include, but not limited to, protic solvents such as water, methanol and ethanol; and aprotic solvents such as pyridine, triethylamine, THF, DMF, diethyl ether, DMSO, dichloromethane, chloroform and toluene. The reaction can be conducted at a temperature in the range of -20 °C to the boiling point of a solvent used.

ii. Reaction Scheme (5)

This synthetic process may be conducted as described for Reaction Scheme (3).

Synthetic method 3

A carboxylic acid or carboxamide compound included in the compounds represented by formula (1) or (2) according to this invention may be prepared as follows.

Synthetic method 3-1

Hydrolysis of the ester group in the compound represented by formula (5a), (5b) or (8) illustrated in synthetic method 1 or 2 under a basic or acidic condition to provide a carboxylic acid derivative represented by formula (9) or (10);

Synthetic method 3-2

Preparation of various amides by a reaction with an appropriate amine in the presence of a condensing agent or by forming an acid halide from the carboxylic acid before reacting with an appropriate amine.

Examples of these synthetic methods are illustrated in Reaction Schemes (6) and (7).

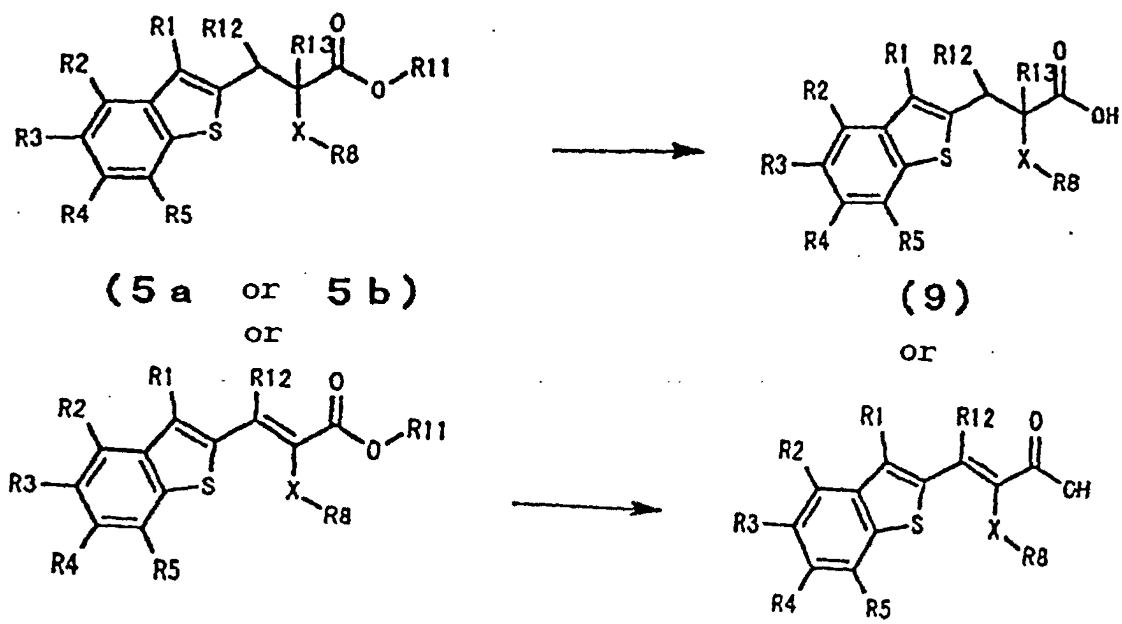

(5a or 5b)

or

(9)

or

(8)

(10)

Reaction Scheme (6)

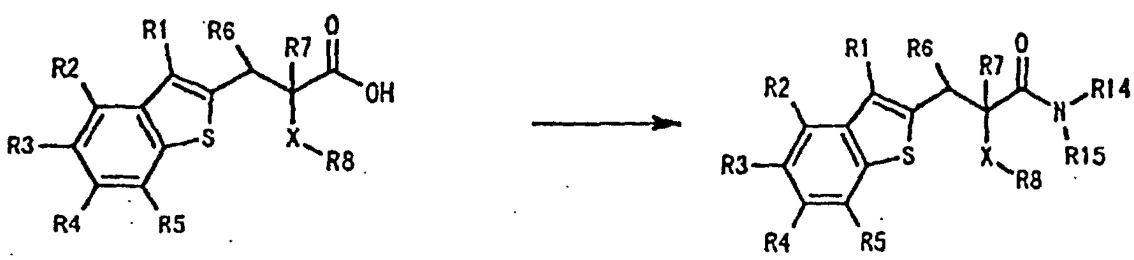

(11)

(12)

Reaction Scheme (7)

wherein $R^1$, $R^2$, $R^3$, $R^4$, $R^5$, $R^6$, $R^7$, $R^8$, $R^{11}$, $R^{12}$, $R^{13}$ and X are as defined above; $R^{14}$ and $R^{15}$ independently represent lower alkyl having 1 to 4 carbon atoms or optionally substituted phenyl.

The reactions according to these equations will be individually described.

i. Reaction Scheme (6)

There are no restrictions to an acid or base used in the synthesis in equation (9) or (10). Examples of acids which can be used in hydrolysis under an acidic condition include, but not limited to, inorganic acids such as hydrochloric acid, sulfonic acid and nitric acid; and organic acids such as trifluoroacetic acid and trifluoromethanesulfonic acid. A solvent may be water or a mixture thereof with one or more appropriate organic solvents. Examples of the appropriate organic solvents include, but not limited to, protic solvents such as methanol and ethanol; and aprotic solvents such as THF, DMF, dioxane and DMSO. There are no restrictions to a mixing ratio of the solvent mixture. The reaction can be conducted at a temperature in the range of 0 °C to the boiling point of a solvent used.

Examples of bases which can be used in hydrolysis under a basic condition include, but not limited to, sodium hydroxide, lithium hydroxide and potassium hydroxide. Examples of solvents which can be used alone include protic solvents such as water, methanol and ethanol; and aprotic solvents such as THF, DMSO, DMF and dioxane.

A solvent may be water or a mixture thereof with one or more appropriate organic solvents. There are no restrictions to the number of the solvent and a mixing ratio of the solvent mixture. Examples of the appropriate organic solvents include, but not limited to, protic solvents such as methanol and ethanol; and aprotic solvents such as THF, DMF, dioxane and DMSO. The reaction can be conducted at a temperature in the range of -20 °C to the boiling point

of a solvent used.

ii. Reaction Scheme (7)

The amide represented by formula (12) can be prepared by an appropriate procedure such as a direct method using a condensing agent and a method via an acid halide.

Examples of condensing agents which can be used in an amidation include, but not limited to, 1, 1-carbonylbisimidazole (hereinafter, referred to as "CDI"), dicyclohexylcarbodiimide (hereinafter, referred to as "DCC"), diethyl cyanophosphate (hereinafter, referred to as "DECP") and mixed acid anhydrides using ethyl chloroformate.

The amide represented by formula (12) may be prepared by derivatizing a compound by using a commonly used acid-halide forming reagent into a corresponding acid halide, which is then converted into the amide by any known amidation.

Synthetic method 4

The compounds represented by formulas (5a) and (5b) and (8) described in synthetic methods 1 and 2 can be converted into a variety of derivatives by an appropriate treatment. Some examples will be described below.

(1) A cyano derivative represented by formula (13) may be converted into an imidate derivative represented by formula (14) by treating it with an acid in an alcohol.

(2) The imidate derivative represented by formula (14) may be further converted into an amidine represented by formula (15).

Examples of these processes are illustrated in Reaction Schemes (8) and (9).

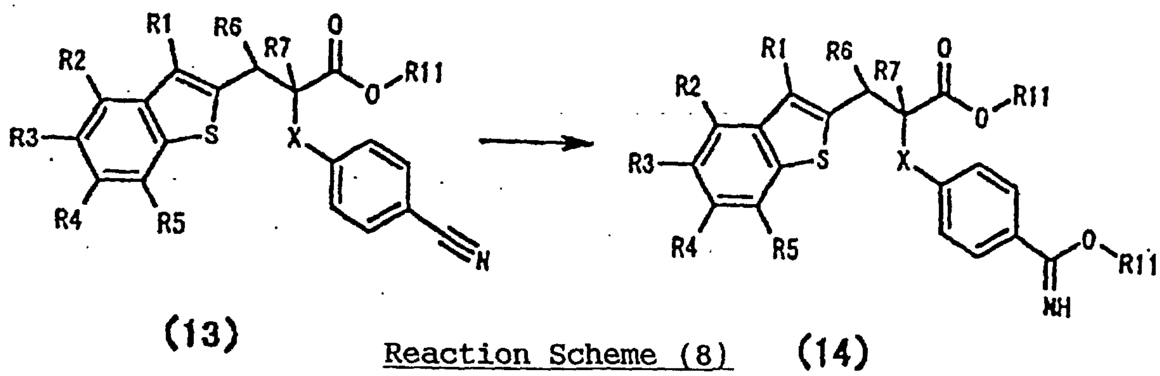

(13) Reaction Scheme (8) (14)

(14)

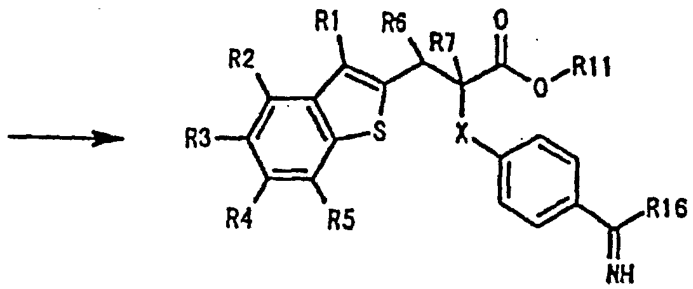

Reaction Scheme (9) (15)

wherein R1, R2, R3, R4, R5, R6, R7, R11 and X are as defined above; R16 represents amino optionally substituted by lower alkyl having 1 to 4 carbon atoms, amino optionally substituted by optionally substituted phenyl or the group represented by formula (16):

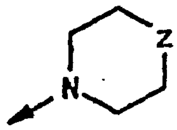 (16)

wherein Z represents oxygen, sulfur or optionally substituted nitrogen.

The reactions according to these equations will be described.

i. Reaction Scheme (8)

Examples of acids which can be used in preparing the imidate represented by formula (14) include, but not limited to, inorganic acids such as hydrochloric acid, sulfonic acid and nitric acid; and organic acids such as trifluoroacetic acid and trifluoromethanesulfonic acid. Examples of solvents include, but not limited to, preferably alcohols such as methanol, ethanol and propanol. The reaction may be conducted at a temperature in the range of -100 °C to the boiling point of a solvent used, preferably 0 °C to room temperature.

ii. Reaction Scheme (9)

A base may be absent or be any base without limitations. Preferably, an excessive amount of a reactant amine may be also used as a base.

Examples of solvents which can be used include, but not limited to, protic solvents such as water, methanol and ethanol; and aprotic solvents such as pyridine, triethylamine, THF, DMF, diethyl ether, DMSO, dichloromethane, chloroform and toluene. The reaction may be conducted at a temperature in the range of -20 °C to the boiling point of a solvent used.

Specific examples of the compounds represented by formula (1) or (2) according to the present invention are as follows, but not limiting in any manner.

(1) methyl 3-(benzothiophen-2-yl)-2-methoxypropanoate;
(2) ethyl 3-(benzothiophen-2-yl)-2-methoxypropanoate;
(3) benzyl 3-(benzothiophen-2-yl)-2-methoxypropanoate;
(4) 4-bromobenzyl 3-(benzothiophen-2-yl)-2-methoxypropanoate;
(5) phenyl 3-(benzothiophen-2-yl)-2-methoxypropanoate;
(6) 2-methoxyphenyl 3-(benzothiophen-2-yl)-2-methoxypropanoate;
(7) 3-(benzothiophen-2-yl)-2-methoxypropanoic acid;
(8) 3-(benzothiophen-2-yl)-2-methoxypropanamide;
(9) 3-(benzothiophen-2-yl)-2-methoxypropanoic acid N-propylamide;
(10) 3-(benzothiophen-2-yl)-2-methoxypropanoic acid N,N-diethylamide;
(11) 3-(benzothiophen-2-yl)-2-methoxypropanoic acid N-phenylamide;
(12) 3-(benzothiophen-2-yl)-2-methoxypropanoic acid N-(3-chlorophenyl)amide;
(13) 3-(benzothiophen-2-yl)-2-methoxy-2-methylpropanoic acid;
(14) 3-(benzothiophen-2-yl)-2-methoxy-2-butylpropanoic acid;
(15) 3-(benzothiophen-2-yl)-2-ethoxy-2-methylpropanoic acid;
(16) 3-(benzothiophen-2-yl)-2-methyl-2-propoxypropanoic acid;
(17) 3-(benzothiophen-2-yl)-2-isopropoxy-2-methylpropanoic acid;
(18) 3-(benzothiophen-2-yl)-2-butoxy-2-methylpropanoic acid;
(19) 3-(benzothiophen-2-yl)-2, 2-diethoxypropanoic acid;
(20) ethyl 3-(benzothiophen-2-yl)-2, 2-diethoxypropanoate;
(21) ethyl 3-(benzothiophen-2-yl)-2-ethoxy-2-methylpropanoate;
(22) 3-(benzothiophen-2-yl)-2-ethoxy-2-methyl-3-methylpropanoic acid;
(23) ethyl 3-(benzothiophen-2-yl)-2-ethoxy-2-methyl-3-methylpropanoate;
(24) 3-(benzothiophen-2-yl)-2-methyl-2-phenylpropanoic acid;
(25) ethyl 3-(benzothiophen-2-yl)-2-methyl-2-phenylpropanoate;
(26) ethyl 3-(benzothiophen-2-yl)-2-methyl-2-(2-methylphenyl)propanoate;
(27) ethyl 3-(benzothiophen-2-yl)-2-(2-chlorophenyl)-2-methylpropanoate;
(28) ethyl 3-(benzothiophen-2-yl)-2-(2-cyanophenyl)-2-methylpropanoate;
(29) ethyl 3-(benzothiophen-2-yl)-2-methyl-2-(3-nitrophenyl)propanoate:

(30) ethyl 2-(3-aminophenyl)-3-(benzothiophen-2-yl)-2-methylpropanoate;
(31) 2-(3-aminophenyl)-3-(benzothiophen-2-yl)-2-methylpropanoic acid;
(32) 2-(4-aminophenyl)-3-(benzothiophen-2-yl)-2-methylpropanoic acid;
(33) 2-(2-aminophenyl)-3-(benzothiophen-2-yl)-2-methylpropanoic acid;
(34) 3-(benzothiophen-2-yl)-2-methyl-2-(4-methylphenyl)propanoic acid;
(35) 3-(benzothiophen-2-yl)-2-(4-ethylphenyl)-2-methylpropanoic acid;
(36) 3-(benzothiophen-2-yl)-2-methyl-2-(4-propylphenyl)propanoic acid;
(37) 3-(benzothiophen-2-yl)-2-(4-isopropylphenyl)-2-methylpropanoic acid;
(38) methyl 3-(benzothiophen-2-yl)-2-(4-isopropylphenyl)-2-methylpropanoate;
(39) ethyl 3-(benzothiophen-2-yl)-2-(4-isopropylphenyl)-2-methylpropanoate;
(40) benzyl 3-(benzothiophen-2-yl)-2-(4-isopropylphenyl)-2-methylpropanoate;
(41) 3-(benzothiophen-2-yl)-2-(4-butylphenyl)-2-methylpropanoic acid;
(42) 3-(benzothiophen-2-yl)-2-(4-isopropylphenyl)-2-methylpropenic acid;
(43) 2-(4-isopropylphenyl)-2-methyl-3-(5-methylbenzothiophen-2-yl)propanoic acid;
(44) 3-(4-ethylbenzothiophen-2-yl)-2-(4-isopropylphenyl)-2-methylpropanoic acid;
(45) 2-(4-isopropylphenyl)-2-methyl-3-(3-propylbenzothiophen-2-yl)propanoic acid;
(46) 3-(7-butylbenzothiophen-2-yl)-2-(4-isopropylphenyl)-2-methylpropanoic acid;
(47) 2-(4-isopropylphenyl)-3-(5-methoxybenzothiophen-2-yl)-2-methylpropanoic acid;
(48) 3-(7-ethoxybenzothiophen-2-yl)-2-(4-isopropylphenyl)-2-methylpropanoic acid;
(49) 3-(6-ethoxybenzothiophen-2-yl)-2-(4-isopropylphenyl)-2-methylpropanoic acid;
(50) 3-(4-butoxybenzothiophen-2-yl)-2-(4-isopropylphenyl)-2-methylpropanoic acid;
(51) methyl 3-(5,6-dimethoxybenzothiophen-2-yl)-2-(4-isopropylphenyl)-2-methylpropanoate;
(52) ethyl 3-(5,6-dimethoxybenzothiophen-2-yl)-2-(4-isopropylphenyl)-2-methylpropanoate;
(53) phenyl 3-(5,6-dimethoxybenzothiophen-2-yl)-2-(4-isopropylphenyl)-2-methylpropanoate;
(54) benzyl 3-(5,6-dimethoxybenzothiophen-2-yl)-2-(4-isopropylphenyl)-2-methylpropanoate;
(55) 3-(5,6-dimethoxybenzothiophen-2-yl)-2-(4-isopropylphenyl)-2-methylpropanoic acid;
(56) methyl 2-(4-cyanophenyl)-3-(5,6-dimethoxybenzothiophen-2-yl)-2-methylpropanoate;
(57) ethyl 2-(4-cyanophenyl)-3-(5,6-dimethoxybenzothiophen-2-yl)-2-methylpropanoate;
(58) 2-(4-cyanophenyl)-3-(4,6-dimethoxybenzothiophen-2-yl)-2-methylpropanoic acid;
(59) 2-(4-cyanophenyl)-3-(4,7-dimethoxybenzothiophen-2-yl)-2-methylpropanoic acid;
(60) 2-(4-cyanophenyl)-3-(5,6-dimethoxybenzothiophen-2-yl)-2-methylpropanoic acid;
(61) 2-(4-cyanophenyl)-3-(5,6-dimethoxybenzothiophen-2-yl)-2-propylpropanoic acid;
(62) 2-(4-cyanophenyl)-3-(5,6-dimethoxybenzothiophen-2-yl)-2-methylpropanamide;
(63) 2-(4-cyanophenyl)-3-(5,6-dimethoxybenzothiophen-2-yl)-2-methylpropanoic acid N-ethylamide;
(64) 2-(4-cyanophenyl)-3-(5,6-dimethoxybenzothiophen-2-yl)-2-methylpropanoic acid N,N-diethylamide;
(65) 2-(4-cyanophenyl)-3-(5,6-dimethoxybenzothiophen-2-yl)-2-methylpropanoic acid N-propylamide;
(66) 2-(4-cyanophenyl)-3-(5,6-dimethoxybenzothiophen-2-yl)-2-methylpropanoic acid N-butylamide;
(67) 2-(4-cyanophenyl)-3-(5,6-dimethoxybenzothiophen-2-yl)-2-methylpropanoic acid N-benzylamide;
(68) 2-(4-cyanophenyl)-3-(5,6-dimethoxybenzothiophen-2-yl)-2-methylpropanoic acid N-phenylamide;
(69) 3-(5-bromobenzothiophen-2-yl)-2-(4-isopropylphenyl)-2-methylpropanoic acid;
(70) methyl 3-(6-chlorobenzothiophen-2-yl)-2-(4-isopropylphenyl)-2-methylpropanoate;
(71) ethyl 3-(7-fluorobenzothiophen-2-yl)-2-(4-isopropylphenyl)-2-methylpropanoate;
(72) 3-(5-aminobenzothiophen-2-yl)-2-(4-isopropylphenyl)-2-methylpropanoic acid;
(73) 3-(6-acetamidebenzothiophen-2-yl)-2-(4-isopropylphenyl)-2-methylpropanoic acid;
(74) 3-(7-benzoylaminobenzothiophen-2-yl)-2-(4-isopropylphenyl)-2-methylpropanoic acid;
(75) 3-[7-(4-chlorobenzoyl)aminobenzothiophen-2-yl]-2-(4-isopropylphenyl)-2-methylpropanoic acid;
(76) 3-(5-butylaminobenzothiophen-2-yl)-2-(4-isopropylphenyl)-2-methylpropanoic acid;
(77) 3-(3-diethylaminobenzothiophen-2-yl)-2-(4-isopropylphenyl)-2-methylpropanoic acid;
(78) 2-(4-isopropylphenyl)-2-methyl-3-(5-nitrobenzothiophen-2-yl)propanoic acid;
(79) 3-(7-hydroxybenzothiophen-2-yl)-2-(4-isopropylphenyl)-2-methylpropanoic acid;
(80) 3-(5,6-dihydroxybenzothiophen-2-yl)-2-(4-isopropylphenyl)-2-methylpropanoic acid;
(81) methyl 3-(4-chloro-5,6-dimethoxybenzothiophen-2-yl)-2-(4-isopropylphenyl)-2-methylpropanoate;
(82) 3-(4-chloro-5,6-dimethoxybenzothiophen-2-yl)-2-(4-isopropylphenyl)-2-methylpropanoic acid;
(83) 3-(4,7-dichloro-5,6-dimethoxybenzothiophen-2-yl)-2-(4-isopropylphenyl)-2-methylpropanoic acid;
(84) 3-(5,6-dimethoxybenzothiophen-2-yl)-2-(4-methoxycarbonylphenyl)-2-methylpropanoic acid;
(85) 3-(5,6-dimethoxybenzothiophen-2-yl)-2-(4-carboxyphenyl)-2-methylpropanoic acid;
(86) 3-(5,6-dimethoxybenzothiophen-2-yl)-2-(4-carbamoylphenyl)-2-methylpropanoic acid;
(87) 3-(5,6-dimethoxybenzothiophen-2-yl)-2-(4-N-methylcarbamoylphenyl)-2-methylpropanoic acid;

(88) 3-(5,6-dimethoxybenzothiophen-2-yl)-2-(4-N,N-dipropylcarbamoylphenyl)-2-methylpropanoic acid;
(89) ethyl 2-(4-amidinophenyl)-3-(5,6-dimethoxybenzothiophen-2-yl)-2-methylpropanoate;
(90) ethyl 3-(5,6-dimethoxybenzothiophen-2-yl)-2-methyl-(4-morpholinoamidinophenyl)propanoate;
(91) 3-(5,6-dimethoxybenzothiophen-2-yl)-2-methyl-(4-morpholinoamidinophenyl)propanoic acid;
(92) 3-(5,6-dimethoxybenzothiophen-2-yl)-2-(4-isopropylphenyl)propenic acid;
(93) ethyl 3-(4-chloro-5,6-dimethoxybenzothiophen-2-yl)-2-(4-isopropylphenyl)propenoate;
(94) 3-(4-chloro-5,6-dimethoxybenzothiophen-2-yl)-2-(4-isopropylphenyl)propenic acid;
(95) ethyl 2-(4-cyanophenyl)-3-(5,6-dimethoxybenzothiophen-2-yl)propenoate;
(96) 2-(4-cyanophenyl)-3-(5,6-dimethoxybenzothiophen-2-yl)propenic acid;
(97) 2-(4-cyanophenyl)-3-(5,6-dimethoxybenzothiophen-2-yl)-3-methylpropenic acid;
(98) methyl 2-(benzothiophen-2-yl)methyl-2-methylmalonamide;
(99) ethyl 2-(benzothiophen-2-yl)methyl-2-methylmalonamide;
(100) 2-(benzothiophen-2-yl)methyl-2-methylmalonamide;
(101) 2-(benzothiophen-2-yl)methyl-2-methylmalonamide sodium salt;
(102) 2-(benzothiophen-2-yl)methyl-2-methylmalonic acid N-methylamide;
(103) 2-(benzothiophen-2-yl)methyl-2-methylmalonic acid N-propylamide;
(104) 2-(benzothiophen-2-yl)methyl-2-methylmalonic acid N,N-diethylamide;
(105) 2-(benzothiophen-2-yl)methyl-2-methylmalonic acid N-cyclohexylamide;
(106) 2-(benzothiophen-2-yl)methyl-2-methylmalonic acid N-morpholinamide;
(107) 2-(benzothiophen-2-yl)methyl-2-methylmalonic acid N-phenylamide;
(108) 2-(benzothiophen-2-yl)methyl-2-methylmalonic acid N-benzylamide;
(109) 2-(benzothiophen-2-yl)methyl-2-methylmalonic acid N-(4-isopropylphenyl)amide;
(110) 2-(benzothiophen-2-yl)methyl-2-methylmalonic acid N-(2-chlorophenyl)amide;
(111) 2-(benzothiophen-2-yl)methyl-2-methylmalonic acid N-(3-methylphenyl)amide;
(112) 2-(5,6-dimethoxybenzothiophen-2-yl)methyl-2-methylmalonamide;
(113) 2-(5,6-dimethoxybenzothiophen-2-yl)methyl-2-ethylmalonamide;
(115) 2-(5,6-dimethoxybenzothiophen-2-yl)methyl-2-isopropylmalonamide;
(116) 2-(5,6-dimethoxybenzothiophen-2-yl)methyl-2-methylmalonic acid N-methylamide;
(117) 2-(5,6-dimethoxybenzothiophen-2-yl)methyl-2-methylmalonic acid N-butylamide;
(118) 2-(5,6-dimethoxybenzothiophen-2-yl)methyl-2-methyimalonic acid N-hexylamide;
(119) 2-(5,6-dimethoxyberizothiophen-2-yl)methyl-2-methylmalonic acid N-cyclohexylamide;
(120) 2-(5,6-dimethoxybenzothiophen-2-yl)methyl-2-methylmalonic acid N,N-dimethylamide;
(121) 2-(5,6-dimethoxybenzothiophen-2-yl)methyl-2-methylmalonic acid N-phenylamide;
(122) 2-(5,6-dimethoxybenzothiophen-2-yl)methyl-2-methylmalonic acid N-(2-fluorophenyl)amide;
(123) 2-(5,6-dimethoxybenzothiophen-2-yl)methyl-2-methylmalonic acid N-(3-bromophenyl)amide;
(124) 2-(5,6-dimethoxybenzothiophen-2-yl)methyl-2-methylmalonic acid N-(4-isopropylphenyl)amide;
(125) 2-(5,6-dimethoxybenzothiophen-2-yl)methyl-2-methylmalonic acid N-benzylamide.

Some of the above compounds have one or more asymmetric carbons and thus exist as optical isomers. All of the optical isomers are, of course, encompassed within this invention. Some of the above compounds exist as stereoisomers derived from one or more carbon-carbon unsaturated bonds, and all of these isomers are also encompassed within this invention.

When using as a medical drug, a compound of the present invention may be administered orally or parenterally. A dose is 1 to 1000 mg per day for an adult, depending on many factors such as symptoms, an age and a sex of the patient, and may be administered as a single dosage or as divided dosages. A dosage form may be administered as an oral preparation such as tablets, granules, powder, capsules, a syrup, a liquid and an emulsion, or a parenteral preparation such as an injection, a suppository and a transdermal form. These dosage forms may contain an absorbent such as crystalline cellulose and light anhydrous silicic acid and an excipient such as corn starch, lactose, calcium phosphate and magnesium stearate, and as necessary, a binder, a wetting agent, a lubricant, a flavoring agent, a coloring agent, a sweetener, a solubilizer and so on.

An injection may be prepared as an isotonic and sterilized aqueous solution; an aqueous suspension containing cottonseed oil, corn oil and olive oil; or an emulsion containing a surfactant such as HCO-60.

This invention will be further described with reference to, but not limited to, Examples and Test Examples.

Example 1

Ethyl 2-(4-cyanophenyl)-3-(5,6-dimethoxybenzothiophen-2-yl)-2-methylpropanoate: Compound (57)

Reaction 1: Synthesis of ethyl 2-(4-cyanophenyl)-3-(5,6-dimethoxybenzothiophen-2-yl)-3-hydroxy-2-methylpropanoate

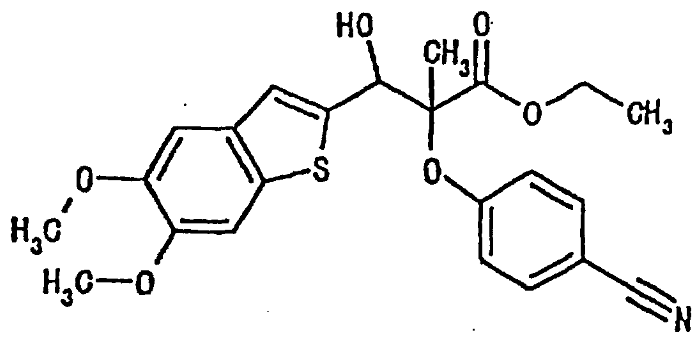

In THF (30 mL) was dissolved isopropylhexylamine (1.98 g, 14 mmol). To the mixture was added dropwise a 1.53M solution of n-butyllithium in hexane (9.15 mL, 14 mmol) at -60 °C, and the mixture was heated to 0 °C and stirred for 20 min at the temperature. The reaction was again cooled to -60 °C, and to the mixture was added dropwise a solution of ethyl 2-(4-cyanophenoxy)propanoate (2.74 g, 12.5 mmol) in THF (20 mL). After stirring at the temperature for one hour, a solution of 5,6-dimethoxybenzothiophen-2-carboxyaldehyde (2.22 g, 10 mmol) in THF (30 mL). After stirring at -55 to -65 °C for 1.5 hours, the reaction was heated to -20 °C and then a saturated brine (30 mL) was added. After adding water (300 mL), the mixture was extracted with ethyl acetate (400 mL). The organic layer was dried over anhydrous magnesium sulfate and evaporated in vacuo. The residue was purified by silica-gel column chromatography (Merck C-300 equivalent, 100 g, ethyl acetate : hexane = 1 : 2 to 1 : 1) to provide the title alcohol compound (3.19 g).

Reaction 2: Synthesis of ethyl 2-(4-cyanophenyl)-3-(5,6-dimethoxybenzothiophen-2-yl)-2-methylpropanoate

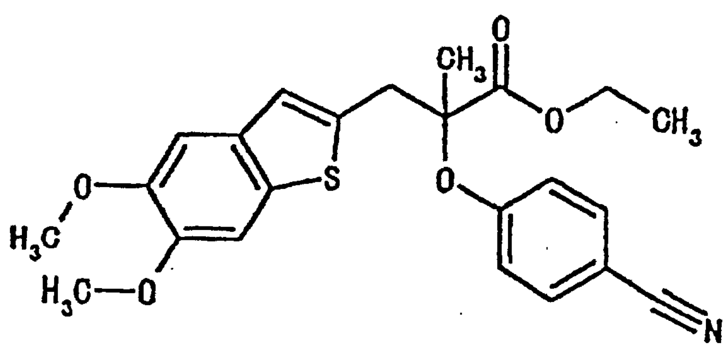

The alcohol compound prepared in step 1 (3.19 g, 7.23 mmol) was dissolved in dichloromethane (45 mL). After adding trifluoroacetic acid (15 mL) and triethylsilane (6.92 mL, 43.4 mL), the mixture was stirred at room temperature for 3 hours. The reaction was poured into a saturated aqueous sodium bicarbonate (300 mL), and the mixture was extracted with dichloromethane (300 mL). The organic layer was dried over anhydrous magnesium sulfate and concentrated under a reduced pressure. The residue was purified by silica-gel column chromatography (Merck C-300 equivalent, 120 g, ethyl acetate : hexane = 1:3 to 1:2) to give the title compound (2.79 g, 66 % / 2 steps) as a colorless and transparent syrup.

$^1$H-NMR ($CDCl_3$, 270 MHz) $\delta$ = 7.57 (d, 2H, J = 8.9 Hz), 7.22 (s, 1H), 7.14 (s, 1H), 6.98 (s, 1H), 6.96 (d, 2H, J =

8.9 Hz), 4.24 (q, 2H, J = 6.9 Hz), 3.93 (s, 6H), 3.60 and 3.44 (2d, each 1H, J = 14.8 Hz), 1.60 (s, 3H), 1.20 (t, 3H, J = 6.9 Hz).

Example 2

Synthesis of 2-(4-cyanophenyl)-3-(5,6-dimethoxybenzothiophen-2-yl)-2-methylpropanoic acid: Compound (60)

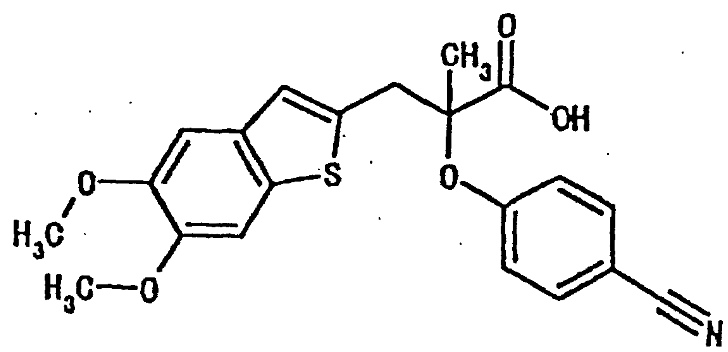

The ester compound prepared in Example 1 (262 mg, 0.623 mmol) was dissolved in ethanol (6 mL). After adding a 2N aqueous solution of sodium hydroxide (0.62 mL, 1.24 mmol), the mixture was stirred at room temperature 24 hours. The reaction was poured into water (50 mL). The mixture was acidified with 1N hydrochloric acid and then extracted with ethyl acetate (100 mL). The organic layer was dried over anhydrous magnesium sulfate and concentrated under a reduced pressure. The residue was crystallized from ethyl acetate and hexane to give the title compound as white crystals (200mg, 81 %).

Melting point = 162 to 164 °C.

$^1$H-NMR (DMSO-$d_6$, 270 MHz) δ = 13.5 (bs, 1H), 7.81 (d, 2H, J = 8.9 Hz), 7.44 (s, 1H), 7.28 (s, 1H), 7.13 (s, 1H), 7.02 (d, 2H, J = 8.9 Hz), 3.79 (s, 6H), 3.57 and 3.47 (2d, each 1H, J = 14.7 Hz), 1.53 (s, 3H).

Example 3

Synthesis of 3-(5,6-dimethoxybenzothiophen-2-yl)-2-(4-isopropylphenoxy)-2-methylpropanoic acid: Compound (55)

Reaction 1: Synthesis of ethyl 3-(5,6-dimethoxybenzothiophen-2-yl)-2-(4-isopropylphenoxy)-2-methylpropanoate

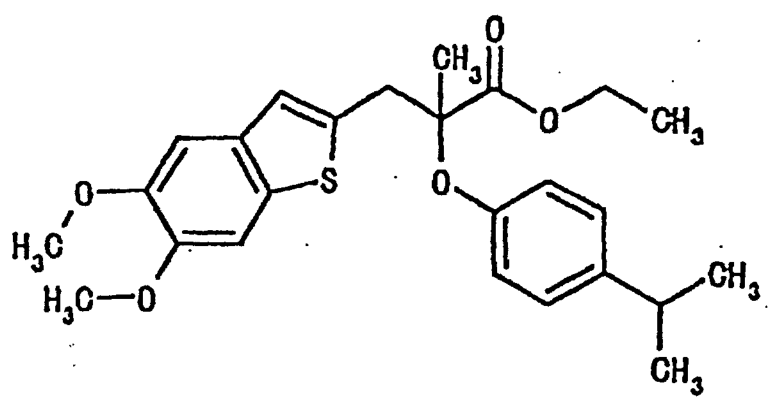

Ethyl 2-(4-isopropylphenoxy)propanoate (1.48 g, 6.25 mmol) and 5,6-dimethoxybenzothiophen-2-carboxyaldehyde (1.10 g, 5 mmol) was treated as described in steps 1 and 2 in Example 1 to give the title compound (960mg, 43 %) as a colorless and transparent syrup.

$^1$H-NMR ($CDCl_3$, 270 MHz) δ = 7.23 - 7.00 (m, 5H), 6.86 (d, 2H, J = 8.6 Hz), 4.25 (q, 2H, J = 7.0 Hz), 3.93 and

3.92 (2s, each 3H), 3.56 and 3.42 (2d, each 1H, J = 14.7 Hz), 2.85 (quintet, 1H, J = 6.7 Hz), 1.48 (s, 3H), 1.25 (t, 3H, J = 7.0 Hz), 1.21 (d, 6H, J = 6.7 Hz).

Reaction 2: Synthesis of 3-(5,6-dimethoxybenzothiophen-2-yl)-2-(4-isopropylphenoxy)-2-methylpropanoic acid

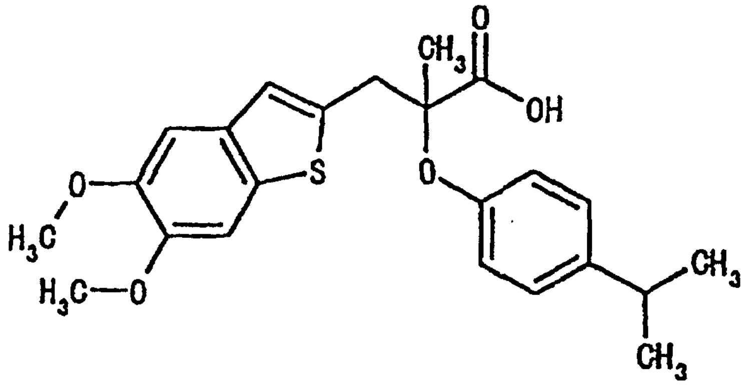

The ester compound prepared in step 1 (950mg, 2.15 mmol) was treated as described in Example 2 to give the title compound (660mg, 74 %) as a white amorphous solid.

$^1$H-NMR (DMSO-$d_6$, 270 MHz) δ = 13.2 (bs, 1H), 7.43 (s, 1H), 7.27 (s, 1H), 7.13 (d, 2H, J = 8.6 Hz), 7.10 (s, 1H), 6.84 (d, 2H, J = 8.6 Hz), 3.80 and 3.79 (2s, each 3H), 3.48 and 3.41 (2d, each 1H, J = 14.8 Hz), 2.82 (quintet, 1H, J = 6.9 Hz), 1.38 (s, 3H), 1.17 (d, 6H, J = 6.9 Hz).

Example 4: Synthesis of 3-(4-chloro-5,6-dimethoxybenzothiophen-2-yl)-2-(4-isopropylphenoxy)-2-methylpropanoic acid: Compound (82)

Reaction 1: Synthesis of ethyl 3-(4-chloro-5,6-dimethoxybenzothiophen-2-yl)-2-(4-isopropylphenoxy)-2-methylpropanoate

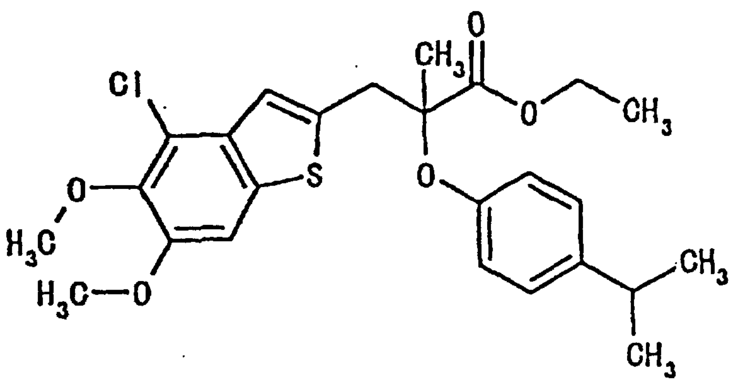

Ethyl 2-(4-isopropylphenoxy)propanoate (1.18 g, 5 mmol) and 4-chloro-5,6-dimethoxybenzothiophen-2-carboxyaldehyde (1.28 g, 5 mmol) were treated as described in steps 1 and 2 in Example 1 to give the title compound (720mg, 30 %) as a colorless and transparent syrup.

$^1$H-NMR ($CDCl_3$, 270 MHz) δ = 7.60 (s, 1H), 7.10 (s, 1H), 7.15 (d, 2H, J = 8.6 Hz), 6.95 (d, 2H, J = 8.6 Hz), 4.25 (q, 2H, J = 7.0 Hz), 3.92 (s, 6H), 3.56 and 3.42 (2d, each 1H, J = 14.5 Hz), 2.85 (quintet, 1H, J = 6.3 Hz), 1.45 (s, 3H), 1.25 (t, 3H, J = 7.0 Hz), 1.20 (d, 6H, J = 6.3 Hz).

Reaction 2: Synthesis of 3-(4-chloro-5,6-dimethoxybenzothiophen-2-yl)-2-(4-isopropylphenoxy)-2-methylpropanoic acid

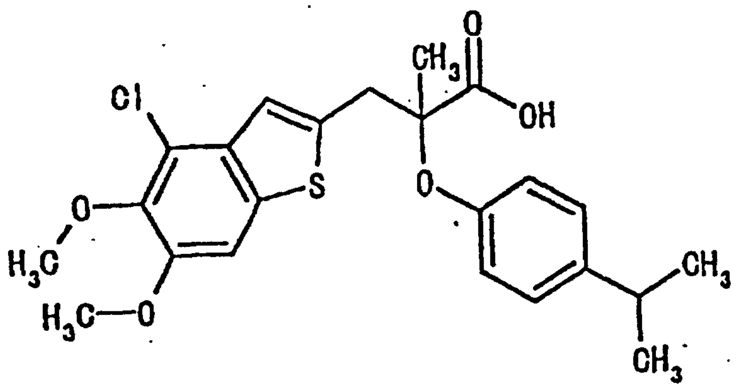

The ester compound prepared in step 1 (710mg, 1.49 mmol) was treated as described in Example 2 to give the title compound (510mg, 76 %) as white crystals.

Melting point = 105 to 108 °C.

$^1$H-NMR (DMSO-$d_6$, 270 MHz) δ = 13.2 (bs, 1H), 7.60 (s, 1H), 7.15 (s, 1H), 7.13 (d, 2H, J = 8.5 Hz), 6.84 (d, 2H, J = 8.5 Hz), 3.80 (s, 6H), 3.48 and 3.40 (2d, each 1H, J = 14.5 Hz), 2.80 (quintet, 1H, J = 6.9 Hz), 1.40 (s, 3H), 1.20 (d, 6H, J = 6.9 Hz).

Example 5 Synthesis of 3-(5,6-dimethoxybenzothiophen-2-yl)-2-methyl-2-(4-morpholinoamidinophenoxy)propanoic acid hydrochloride: Compound (91)

Reaction 1: Synthesis of ethyl 3-(4-chloro-5,6-dimethoxybenzothiophen-2-yl)-2-methyl-2-(4-morpholinoamidinophenoxy)methylpropanoate

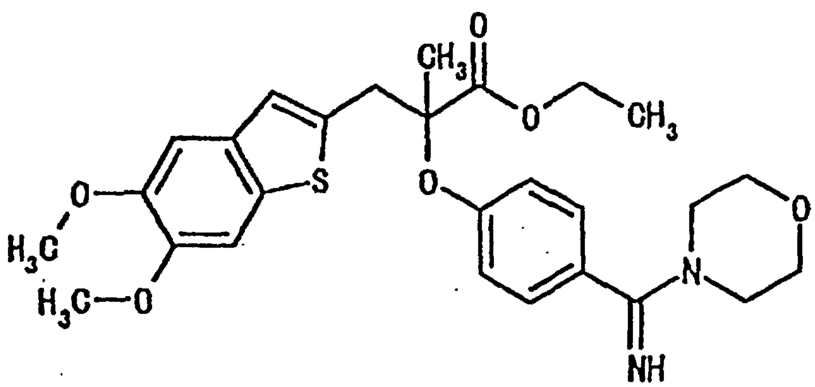

The cyano compound prepared in Example 1 (310 g, 0.729 mmol) was dissolved in ethanol (15 mL). To the mixture at 0 °C was bubbled HCl gas until saturation. The reaction solution was warmed to room temperature and stirred at the temperature for 3 hours. After concentrating under a reduced pressure, the residue was again dissolved in ethanol (10 mL). To the solution was added morpholine (380 mg, 4.38 mmol), and the mixture was stirred at room temperature for 2 hours. After concentrating the reaction under a reduced pressure, the residue was diluted with ethyl acetate and the solution was washed with water. The product was purified by silica-gel column chromatography (Merck C-300 equivalent, 10 g, ethyl acetate : hexane = 1 : 3 to ethyl acetate : chloroform = 1 : 5) to give the title compound (365mg, 98 %) as a colorless and transparent syrup. The title compound (720 mg, 30%) was produced as a amorphous solid.

$^1$H-NMR ($CDCl_3$, 270 MHz) δ =7.29-7.23 (s, 3H), 7.14 (s, 1H), 7.00 (s, 1H), 6.93 (d, 2H, J = 8.6 Hz), 4.26 (q, 2H,

J = 7.3 Hz), 3.94 and 3.93 (s, each 3H), 3.73-3.70 (m, 4H), 3.59 and 3.43 (2d, each 1H, J = 14.8 Hz), 3.63 - 3.32 (m, 4H), 1.55 (s, 3H), 1.24 (t, 3H, J = 7.3 Hz).

Reaction 2: Synthesis of 3-(5,6-dimethoxybenzothiophen-2-yl)-2-methyl-2-(4-morpholinoamidinophenoxy)propanoic acid hydrochloride

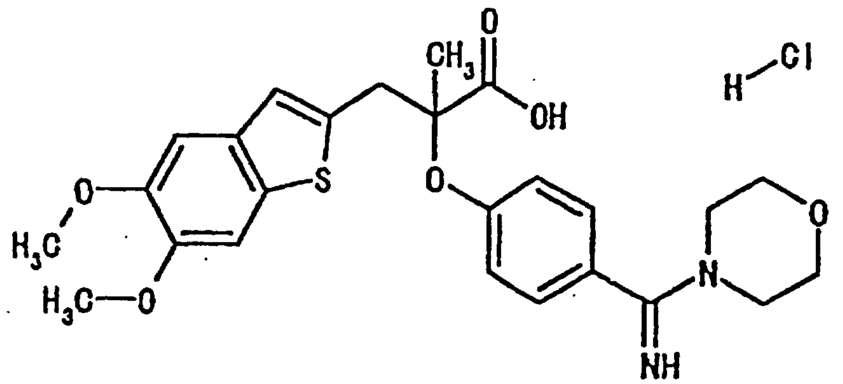

The ester compound prepared in step 1 (360mg, 0.702 mmol) was dissolved in THF (7 mL) and water (7 mL). After adding lithium hydroxide monohydrate (84mg, 1.40 mmol), the mixture was stirred at room temperature for 24 hours. Most of THF was removed from the reaction solution, and the residual solution was acidified to pH = 2 to 3 with 1N hydrochloric acid, to precipitate white crystals. The precipitated crystals were collected by filtration (244 mg) and then treated with 4N hydrochloric acid - dioxane to give the title compound (210mg, 55 %) as white crystals.

Melting point = 115 to 117 °C.

$^1$H-NMR (DMSO-$d_6$, 270 MHz) δ = 13.4 (bs, 1H), 7.82 (d, 2H, J = 8.9 Hz), 7.45 (s, 1H), 7.28 (s, 1H), 7.12 (s, 1H), 6.91 (d, 2H, J = 8.9 Hz), 3.79 (s, 6H), 3.55 and 3.45 (2d, each 1H, J = 14.8 Hz), 3.32 (bs, 8H), 1.48 (s, 3H).

Example 6 Synthesis of (Z)-3-(4-chloro-5,6-dimethoxybenzothiophen-2-yl)-2-(4-isopropylphenoxy)propenoic acid: Compound (94)

Reaction 1: Synthesis of ethyl (Z)-3-(4-chloro-5,6-dimethoxybenzothiophen-2-yl)-2-(4-isopropylphenoxy)propenoate

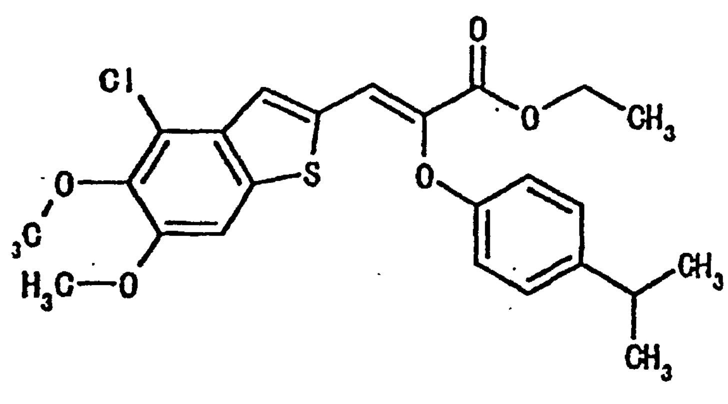

In ethanol (20 mL) were dissolved Ethyl (4-isopropylphenoxy)acetate (533mg, 2.4 mmol) and 4-chloro-5,6-dimethoxybenzothiophen-2-carboxyaldehyde (513mg, 2 mmol). After adding 1 N ethanolic sodium ethoxide (2.4 mL), the mixture was stirred at 60 °C for 4 hours. The reaction mixture was poured into water (150 mL), and the resulting mixture was extracted with ethyl acetate (150 mL). The organic layer was dried over anhydrous magnesium sulfate and then concentrated under a reduced pressure. The product was purified by silica-gel column chromatography (Merck C-300 equivalent, 50 g, ethyl acetate : hexane = 1 : 3) to give the title compound (433mg, 47 %) as a pale yellow syrup.

$^1$H-NMR ($CDCl_3$, 270 MHz) δ = 7.75 (s, 1H), 7.55 (s, 1H), 7.25 - 6.85 (m, 5H), 4.20 (q, 2H, J = 7.0 Hz), 3.90 (s, 6H), 2.85 (quintet, 1H, J = 6.5 Hz), 1.25 (t, 3H, J = 7.0 Hz), 1.20 (d, 6H, J = 6.5 Hz).

Reaction 2: Synthesis of (Z)-3-(4-chloro-5,6-dimethoxybenzothiophen-2-yl)-2-(4-isopropylphenoxy)propenoic acid

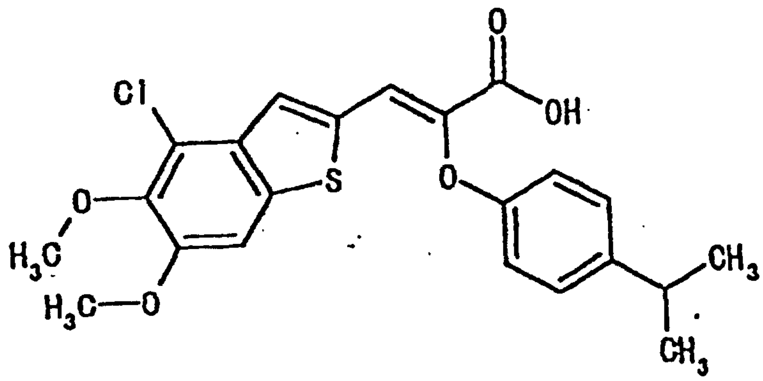

The ester prepared in step 1 (400mg, 0.868 mmol) was treated as described in Example 2 to give the title compound (325mg, 87 %) as white crystals.

Melting point = 255 to 256 °C.

$^1$H-NMR (DMSO-$d_6$, 270 MHz) δ = 13.2 (bs, 1H), 7.75 (s, 1H), 7.50 (s, 1H), 7.30 - 6.80 (m, 5H), 3.85 (s, 6H), 2.85 (quintet, 1H, J = 6.5 Hz), 1.20 (d, 6H, J = 6.5 Hz).

Example 7 Synthesis of(Z)-2-(4-cyanophenoxy)-3-(5,6-dimethoxybenzothiophen-2-yl)propenoic acid: Compound (96)

Reaction 1: Synthesis of ethyl (Z)2-(4-cyanophenoxy)-3-(5,6-dimethoxybenzothiophen-2-yl)propenoate

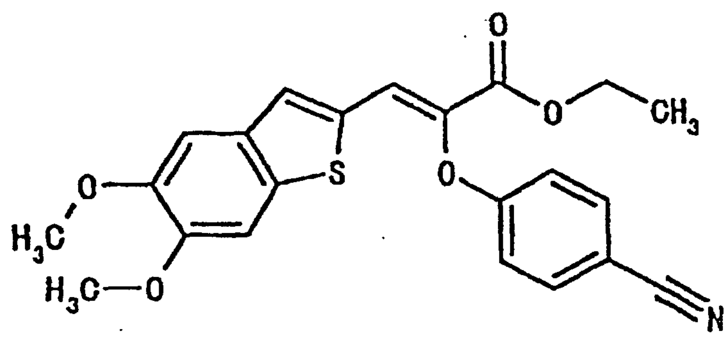

Ethyl (4-cyanophenoxy)acetate (618mg, 3 mmol) and 5,6-dimethoxybenzothiophen-2-carboxyaldehyde (446mg, 2 mmol) were treated as described in step 1 in Example 6 to give the title compound (258mg, 31 %) as an yellow amorphous solid.

$^1$H-NMR ($CDCl_3$, 90 MHz) δ = 7.76 (s, 1H), 7.63 (dd, 2H, J = 2.2, 8.8 Hz), 7.46 (s, 1H), 7.30 (s, 1H), 7.21 - 7.01 (m, 3H), 7.16 (s, 1H), 4.23 (q, 2H, J = 7.1 Hz), 3.91 (s, 6H), 1.25 (t, 3H, J = 7. Hz).

Reaction 2: Synthesis of (Z)-2-(4-cyanophenoxy)-3-(5,6-dimethoxybenzothiophen-2-yl)propenoic acid

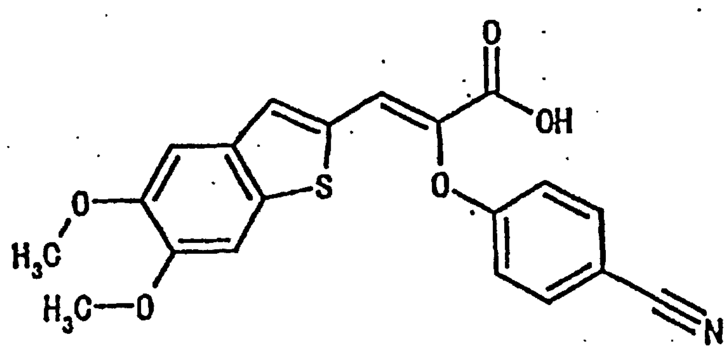

The ester compound prepared in step 1 (251mg, 0.61 mmol) was treated as described in Example 2 to give the title compound (192mg, 83 %) as white crystals.

Melting point = 212 °C (dec.).

$^1$H-NMR ($CDCl_3$, 270 MHz) δ = 13.2 (bs, 1H), 7.87 (s, 1H), 7.63 (dd, 2H, J = 2.0, 6.9 Hz), 7.48 (s, 1H), 7.17 (s, 1H), 7.15 (s, 1H), 7.12 (dd, 2H, J = 2.0, 6.9 Hz), 4.54 (bs, 1H), 3.93 and 3.92 (2s, each 3H).

Example 8

Synthesis of 2-(benzothiophen-2-yl)methyl-2-methylmalonamide ethyl ester: Compound (99)

Reaction 1: Synthesis of diethyl 2-(benzothiophen-2-yl)methyl-2-methylmalonate

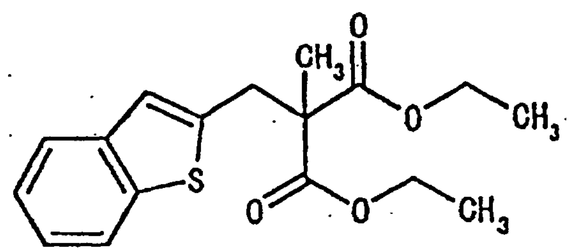

Diethyl methylmalonate (2.61 g, 15 mmol) was dissolved in DMF (40 mL). To the solution at room temperature was added sodium hydride (600mg, 15 mmol) and the mixture was stirred for one hour. Then, to the mixture was added dropwise a solution of benzothiophen-2-yl)methylbromide (2.27 g, 10 mmol) in DMF (20 mL). The reaction was stirred at room temperature for additional two hours. The reaction was poured into water (200 mL) and the product was extracted with ethyl acetate. The extract was washed with water three times. The organic layer was dried over anhydrous magnesium sulfate, and then concentrated under a reduced pressure. The resulting product was purified by silica-gel column chromatography(Merck C-300 equivalent, 120 g, ethyl acetate : hexane = 1 : 15) to give the title compound (3.18 g, 99 %) as a colorless and transparent syrup.

$^1$H-NMR ($CDCl_3$, 270 MHz) δ = 7.79-7.62 (m, 2H), 7.38-7.20 (m, 2H), 7.02 (s, 1H), 4.24 (q, 4H, J = 7.0 Hz), 3.51 (s, 2H), 1.45 (s, 3H), 1.27 (t, 6H, J = 7.0 Hz)

Reaction 2: Synthesis of monoethyl 2-(benzothiophen-2-yl)methyl-2-methylmalonate

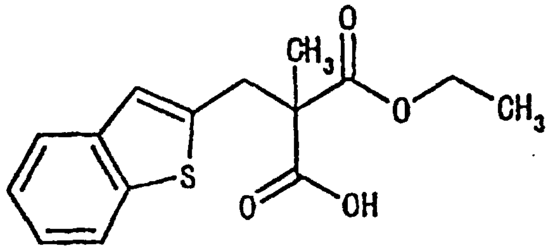

The diester compound prepared in step 1 (3.18 g, 9.94 mmol) was dissolve in ethanol (50 mL). To the solution at room temperature was added a 2 N aqueous solution of sodium hydroxide (5 mL, 10 mmol), and the mixture was stirred at room temperature for 6 hours. After evaporating ethanol, water (200 mL) was added to the mixture. After acidifying by adding 1 N hydrochloric acid, the mixture was extracted with ethyl acetate. The organic layer was dried over anhydrous magnesium sulfate and concentrated under a reduced pressure. The product was purified by silica-gel column chromatography (Merck C-300 equivalent, 120 g, methanol : chloroform = 1 : 15) to give the title compound (2.65 g, 90 %) as a colorless and transparent syrup.

$^1$H-NMR ($CDCl_3$, 270 MHz) $\delta$ = 7.72 (d, 1H, J = 8.5 Hz), 7.65 (dd, 1H, J = 1.7, 8.5 Hz), 7.36 - 7.06 (m, 2H), 7.06 (s, 1H), 5.16 (bs, 1H), 4.25 (q, 2H, J = 7.3 Hz), 3.63 and 3.46 (2d, each 1H, J = 14.7 Hz), 1.51 (s, 3H), 1.27 (t, 3H, J = 7.0 Hz).

Reaction 3: Synthesis of 2-(benzothiophen-2-yl)methyl-2-methylmalonamide ethyl ester

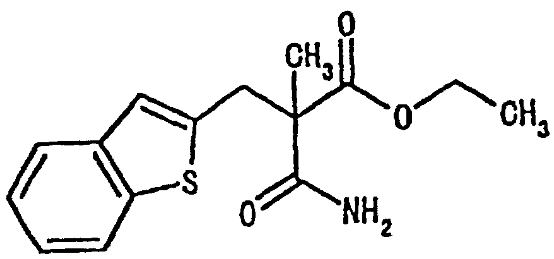

The monoester compound prepared by step 2 (889mg, 3 mmol) was dissolved in DMF (15 mL). To the solution was 1,1-carbonylbisimidazole (648mg, 4 mmol), and the mixture was stirred at room temperature for 1 hour. Then, 28 % ammonia water (5 mL) was added and the mixture was stirred for 1 hour. The reaction was poured into water (50 mL), and the mixture was extracted with ethyl acetate. The extract was sequentially washed with 1N hydrochloric acid and saturated brine. The organic layer was dried over anhydrous magnesium sulfate and concentrated under a reduced pressure. The product was crystallized from ethyl acetate and hexane to give the title compound (730mg, 84 %) as white crystals.

Melting point = 141 to 143 °C.

$^1$H-NMR ($CDCl_3$, 270 MHz) $\delta$ = 7.74 (d, 1H, J = 8.0 Hz), 7.67 (d, 1H, J = 8.3 Hz), 7.34 - 7.23 (m, 2H), 7.06 (s, 1H), 6.94 and 5.52 (bs, each 1H), 4.26 (q, 2H, J = 7.3 Hz), 3.68 and 3.42 (2d, each 1H, J = 14.5 Hz), 1.54 (s, 3H), 1.33 (t, 3H, J = 7.3 Hz).

Example 9

Synthesis of 2-(benzothiophen-2-yl)methyl-2-methylmalonamide sodium salt: Compound (101)

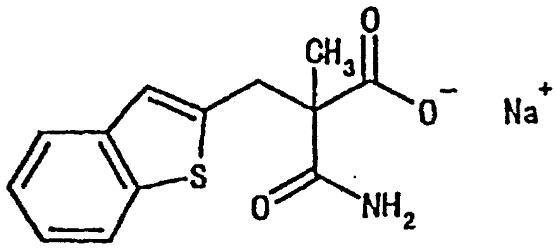

The amide compound prepared in Example 8 (500mg, 1.72 mmol) was dissolved in ethanol (15 mL) and THF (5 mL). To the solution was added an aqueous 2 N sodium hydroxide solution (1.72 mL, 3.44 mmol), and the mixture was stirred at room temperature for 2 hours. The precipitated white crystals were collected by filtration and washed with a small amount of ethanol to give the title compound (430 mg, 88 %).

Melting point = > 230 °C (dec.).

$^1$H-NMR (DMSO-$d_6$ + $D_2O$ 270 MHz) δ = 7.78 (d, 1H, J = 7.5 Hz), 7.66 (d, 1H, J = 7.5 Hz), 7.29-7.18 (m, 2H), 7.07 (s, 1H), 3.28 (s, 2H), 1.67 (s, 3H).

Example 10

Synthesis of 2-(benzothiophen-2-yl)methyl-2-methylmalonic acid N-cyclohexylamide: Compound (105)

Reaction 1: Synthesis of 2-(benzothiophen-2-yl)methyl-2-methylmalonic acid N-cyclohexylamide ethyl ester

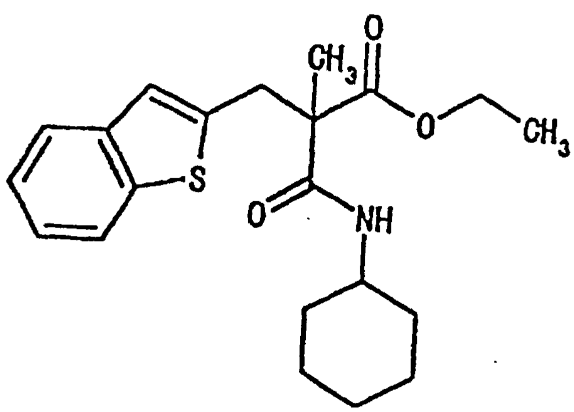

The carboxylic acid compound prepared in step 2 in Example 8 (593mg, 2 mmol) was dissolved in DMF (10 mL). To the solution was added 1,1-carbonylbisimidazole (486 mg, 3 mmol), and the mixture was stirred at room temperature for 1.5 hours. After adding cyclohexylamine (595 mg, 6 mmol), the mixture was stirred at room temperature for 6 hours. The precipitated urea derivative was filtered off. The filtrate was dissolved in ethyl acetate (100 mL) and washed with water three times. The organic layer was dried over anhydrous magnesium sulfate and concentrated under a reduced pressure. The product was purified by silica-gel column chromatography (Merck C-300 equivalent, 50 g, ethyl acetate : hexane = 1 : 7) to give the title compound (510mg, 68 %) as a colorless and transparent syrup.

$^1$H-NMR ($CDCl_3$, 270 MHz) δ = 7.73 (d, 1H, J = 8.0 Hz), 7.65 (d, 1H, J = 8.0 Hz), 7.30 - 7.23 (m, 2H), 7.03 (s, 1H), 6.78 (bd, 1H, J = 7.6 Hz), 4.24 (q, 2H, J = 7.3 Hz), 3.81 - 3.74 (m, 1H), 3.64 and 3.41 (2d, each 1H, J = 14.5 Hz), 1.80 - 1.10 (m, 10H), 1.49 (s, 3H), 1.32 (t, 3H, J = 7.3 Hz).

Reaction 2: Synthesis of 2-(benzothiophen-2-yl)methyl-2-methylmalonic acid N-cyclohexylamide

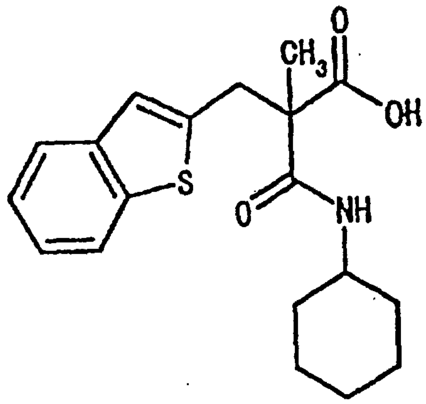

The ester compound prepared in step 1 (490 mg, 1.31 mmol) was treated as described in Example 2 to give the title compound (430 mg, 95 %) as white crystals.

Melting point = 164 °C (dec.).

$^1$H-NMR (DMSO-$d_6$, 270 MHz) δ = 12.8 (bs, 1H), 7.83 (d, 1H, J = 8.0 Hz), 7.72 (d, 1H, J = 8.0 Hz), 7.51 (d, 1H, J = 7.9 Hz), 7.33 - 7.23 (m, 2H), 7.13 (s, 1H), 3.70-3.50 (m, 1H), 3.47 and 3.35 (2s, each 1H, J = 14.5 Hz), 1.80 - 1.50 (m, 5H), 1.30 - 1.00 (m, 5H), 1.28 (s, 3H).

Example 11

Synthesis of 2-(benzothiophen-2-yl)methyl-2-methylmalonic acid N-morpholinoamide: Compound (106)

Reaction 1: Synthesis of 2-(benzothiophen-2-yl)methyl-2-methylmalonic acid N-morpholinoamide ethyl ester

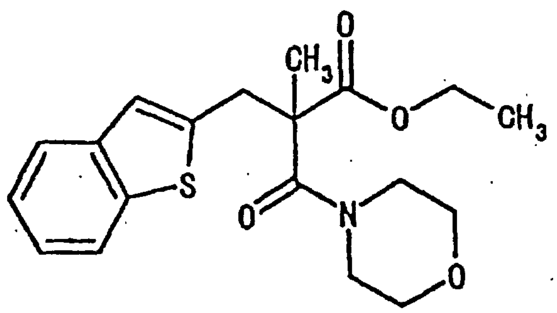

The carboxylic acid compound prepared in step 2 in Example 8 (593 mg, 2 mmol) and morpholine (871 mg, 10 mmol) were treated as described in step 1 in Example 10 to give the title compound (385 mg, 53 %) as a colorless and transparent syrup.

$^1$H-NMR ($CDCl_3$, 270 MHz) δ = 7.74 (d, 1H, J = 8.0 Hz), 7.65 (d, 1H, J = 8.0 Hz), 7.33 - 7.23 (m, 2H), 7.03 (s, 1H), 4.16 (q, 2H, J = 7.0 Hz), 3.70 - 3.40 (m, 8H), 3.59 and 3.42 (2d, each 1H, J = 14.6 Hz), 1.50 (s, 3H), 1.20 (t, 3H, J = 7.0 Hz).

Reaction 2: Synthesis of 2-(benzothiophen-2-yl)methyl-2-methylmalonic acid N-morpholinoamide

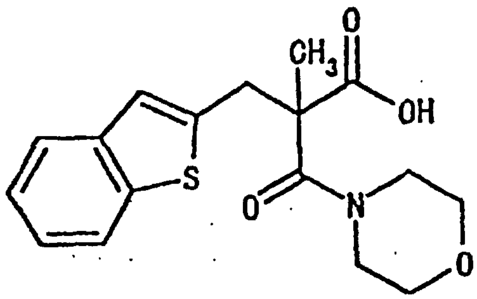

The ester compound prepared in step 1 (370 mg, 1.02 mmol) was treated as described in Example 2 to give the title compound (280 mg, 82 %) as white crystals.

Melting point = 158 °C (dec.).

$^1$H-NMR (DMSO-$d_6$, 270 MHz) δ = 7.74 (d, 1H, J = 8.0 Hz), 7.65 (d, 1H, J = 8.0 Hz), 7.33 - 7.23 (m,2H), 7.02 (s, 1H), 3.70 - 3.50 (m, 8H), 3.59 and 3.40 (2d, each 1H, J = 14.5 Hz), 1.52 (s, 3H).

Example 12

Synthesis of 2-(benzothiophen-2-yl)methyl-2-methylmalonic acid N-(4-isopropylphenyl)amide: Compound (109)

Reaction 1: Synthesis of 2-(benzothiophen-2-yl)methyl-2-methylmalonic acid N-(4-isopropylphenyl)amide ethyl ester

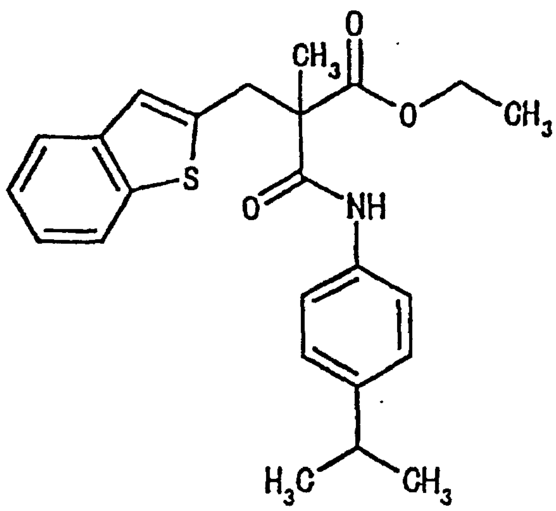

The carboxylic acid compound prepared in step 2 in Example 8 (510mg, 1.72 mmol) was dissolved in DMF (10 mL). To the solution at room temperature was added 1,1-carbonylbisimidazole (363 mg, 2.24 mmol), and the mixture was stirred for 1.5 hours. To the solution at room temperature was slowly added a solution prepared by dissolving 4-isopropylaniline (871 mg, 10 mmol in DMF (5 mL), adding sodium hydride (140mg, 3.44 mmol) and then heating the mixture at 60 °C for 30 min. The reaction was stirred at room temperature for 2 hours, and the mixture was poured into water (150 mL). The resulting mixture was extracted with ethyl acetate (150 mL). The organic layer was washed with water three times, dried over anhydrous magnesium sulfate and concentrated under a reduced pressure. The product was purified by silica-gel column chromatography (Merck C-300 equivalent, 20 g, ethyl acetate : hexane = 1 : 12) to give the title compound (260 mg, 37 %) as a colorless and transparent syrup.

$^1$H-NMR (DMSO-$d_6$, 270 MHz) δ = 9.59 (s, 1H), 7.86 (d, 1H, J = 8.0 Hz), 7.75 (d, 1H, J = 8.0 Hz), 7.50 (d, 2H, J

= 8.5 Hz), 7.35 - 7.24 (m, 2H), 7.21 (s, 1H), 7.18 (d, 2H, J = 8.5 Hz), 4.16 (q, 2H, J = 7.3 Hz), 3.64 and 3.52 (2d, each 1H, J = 14.7 Hz), 2.85 (quintet, 1H, J = 6.6 Hz), 1.44 (s, 3H), 1.19 (d, 6H, J = 6.6 Hz), 1.18 (t, 3H, J = 7.3 Hz).

Reaction 2: Synthesis of 2-(benzothiophen-2-yl)methyl-2-methylmalonic acid N-(4-isopropylphenyl)amide

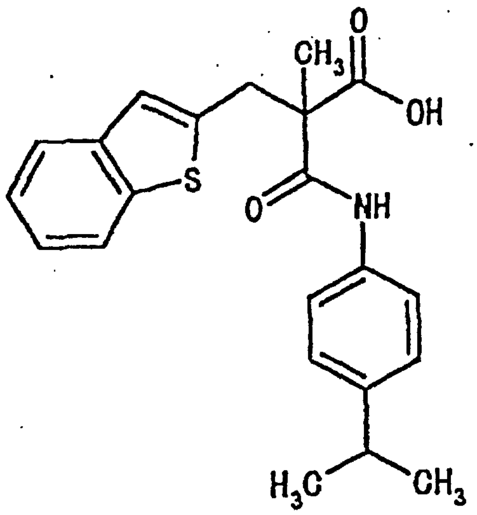

The ester compound prepared in step 1 (180 mg, 0.439 mmol) was treated as described in Example 2 to give the title compound (138 mg, 82 %) as white crystals.

Melting point = 178 °C (dec.).

$^1$H-NMR (DMSO-$d_6$, 270 MHz) δ = 13.01 (bs, 1H), 9.57 (s, 1H), 7.84 (d, 1H, J = 8.0 Hz), 7.74 (d, 1H, J = 8.0 Hz), 7.53 (d, 2H, J = 8.4 Hz), 7.34 - 7.23 (m, 2H), 7.19 (s, 1H), 7.18 (d, 2H, J = 8.4 Hz), 3.66 and 3.46 (2d, each 1H, J = 14.3 Hz), 2.85 (quintet, 1H, J = 7.0 Hz), 1.42 (s, 3H), 1.19 (d, 6H, J = 7.0 Hz).

Example 13

Synthesis of 2-(5,6-dimethoxybenzothiophen-2-yl)methyl-2-methylmalonamide: Compound (112)

Reaction 1: Synthesis of monoethyl 2-(5,6-dimethoxybenzothiophen-2-yl)methyl-2-methylmalonate

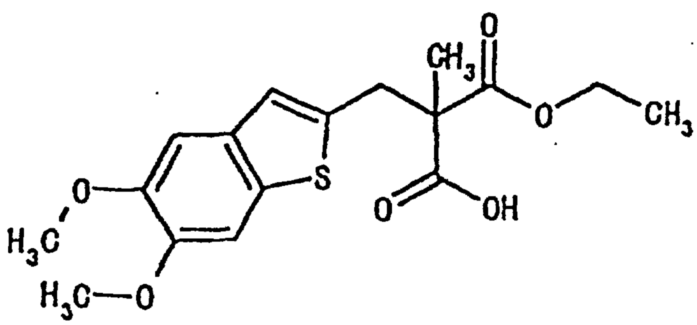

(5,6-Dimethoxybenzothiophen-2-yl)methanol (580 mg, 2.4 mmol) was dissolved in dichloromethane (6 mL). To the solution was added 47 % hydrobromic acid (4.5 mL) and the mixture was stirred at room temperature for 1.5 hours. The reaction was poured into water (50 mL). The resulting mixture was extracted with chloroform (20 mL). The resulting solution of (5,6-dimethoxybenzothiophen-2-yl)methylbromide and diethyl 2-methylmalonate (540 mg, 3.1 mmol) were treated as described in steps 1 and 2 in Example 8 to give the title compound (589 mg, 71 % / 2 steps)

as white crystals.

Melting point = 134 to 137 °C.

$^1$H-NMR ($CDCl_3$, 270 MHz) δ = 7.18 (s, 1H), 7.12 (s, 1H), 6.95 (s, 1H), 4.28 (q, 2H, J = 7.0 Hz), 3.91 (s, 6H), 3.57 and 3.44 (2d, each 1H, J = 15 Hz), 1.52 (s, 3H), 1.32 (t, 3H, J = 7.0 Hz).

Reaction 2: Synthesis of 2-(5,6-dimethoxybenzothiophen-2-yl)methyl-2-methylmalonamide ethyl ester

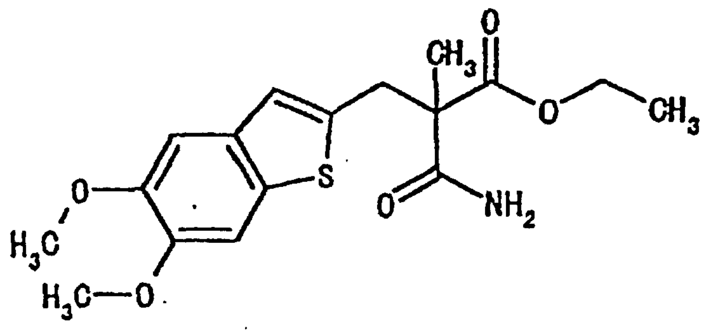

The carboxylic acid compound prepared in step 1 (128 mg, 0.36 mmol) was treated as described in step 3 in Example 8 to give the title compound (120 mg, 94 %) as white crystals.

Melting point = 108 to 110 °C.

$^1$H-NMR ($CDCl_3$, 90 MHz) δ = 7.18 (s, 1H), 7.11 (s, 1H), 6.93 (s, 1H), 4.26 (q, 2H, J = 7.0 Hz), 3.91 (s, 6H), 3.82 and 3.35 (2d, each 1H, J = 15 Hz), 1.91 (bs, 2H), 1.53 (s,-3H), 1.33 (t, 3H, J = 7.0 Hz).

Reaction 3: Synthesis of 2-(5,6-dimethoxybenzothiophen-2-yl)methyl-2-methylmalonic acid

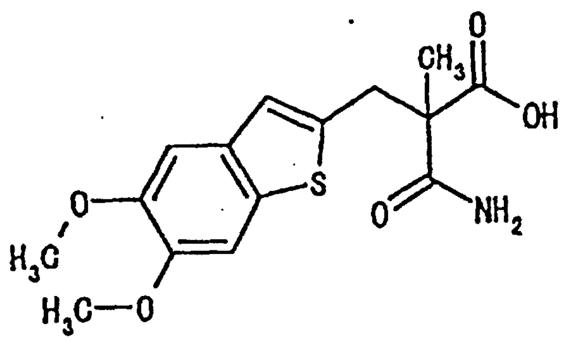

The ester compound prepared in step 1 (117 mg, 0.33 mmol) was treated as described in Example 2 to give the title compound (80 mg, 76 %) as white crystals.

Melting point = 171 to 172 °C.

$^1$H-NMR (DMSO-$d_6$, 270 MHz) δ = 7.41 (s, 1H), 7.34 (s, 1H), 7.26 (s, 1H), 7.22 (s, 1H), 7.00 (s, 1H), 3.79 and 3.78 (2s, each 3H), 3.33 (s, 2H), 1.26 (s, 3H).

Example 14

Synthesis of 2-(5,6-dimethoxybenzothiophen-2-yl)methyl-2-methylmalonic acid N-cyclohexylamide: Compound (119)

Reaction 1: Synthesis of 2-(5,6-dimethoxybenzothiophen-2-yl)methyl-2-methylmalonic acid N-cyclohexylamide ethyl ester

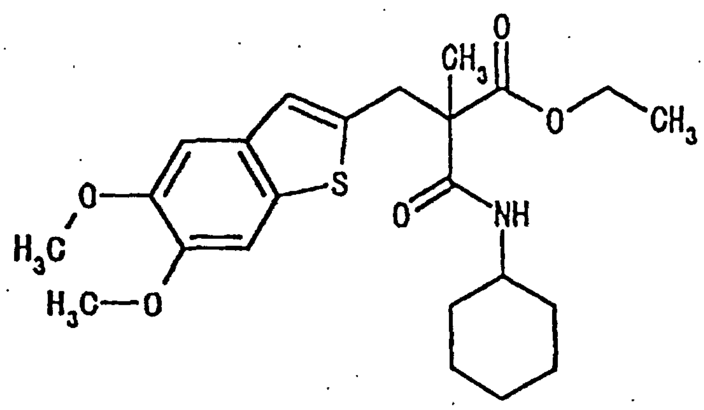

The carboxylic acid compound prepared in step 1 in Example 13 (580 mg, 1.6 mmol) and cyclohexylamine (0.3 mL, 2.6 mmol) were treated as described in step 1 in Example 10 to give the title compound (577 mg, 1.3 mmol) as a white amorphous solid.

$^1$H-NMR ($CDCl_3$, 270 MHz) δ = 7.18 (s, 1H), 7.09 (s, 1H), 6.91 (s, 1H), 6.75 (d, 1H, J = 8.1 Hz), 4.25 (q, 2H, J = 7.0 Hz), 3.92 and 3.91 (2s, each 3H), 3.80 - 3.77 (m, 1H9, 3.46 and 3.36 (2d, each 1H, J = 15 Hz), 1.91 to 1.75 (m, 2H), 1.53 (s, 3H), 1.33 (t, 3H, J = 7.0 Hz).

Reaction 2: Synthesis of 2-(5,6-dimethoxybenzothiophen-2-yl)methyl-2-methylmalonic acid N-cyclohexylamide

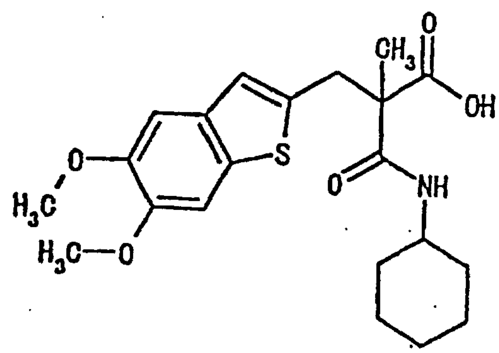

The ester compound prepared in step 1 (571 mg, 1.3 mmol) was treated as described in Example 2 to give the title compound (421 mg, 77 %) as white crystals.

Melting point = 134 to 135 °C.

$^1$H-NMR ($CDCl_3$, 270 MHz) δ = 7.18 (s, 1H), 7.10 (s, 1H), 6.95 (s, 1H), 6.10 (d, 1H, J = 8.6 Hz), 3.92 and 3.91-(2s, each 3H), 3.83 - 3.72 (m, 1H), 3.65 and 3.26 (2d, each 1H, J = 15 Hz), 1.94 to 1.66 (m, 2H), 1.66 - 1.50 (m, 1H, 1.58 (s, 3H), 1.45 - 1.19 (m, 2H9, 1.19 - 0.91 (m, 3H).

Example 15

Synthesis of 2-(4-cyanophenyl)-3-(5,6-dimethoxybenzothiophen-2-yl)-2-methylpropanamide: Compound (62)

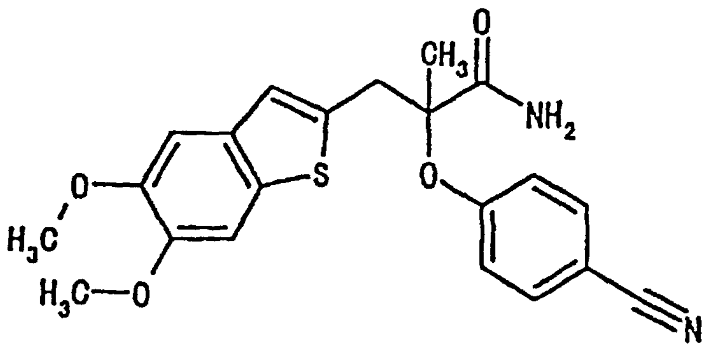

The carboxylic acid compound prepared in Example 2 (560 mg, 1.41 mmol) was treated as described in Example 9 to give the title compound (460 mg, 82 %) as white crystals.

Melting point = 178 to 180 °C.

$^1$H-NMR ($CDCl_3$, 270 MHz) δ = 7.61 (d, 2H, J = 8.8 Hz), 7.21 (s, 1H), 7.13 - 7.09 (m, 3H), 6.99 (s, 1H), 6.29 and 5.68 (sbs, each 1H), 3.93 and 3.92 (2s, each 3H), 3.58 and 3.45 (2d, each 1H, J = 15.4 Hz), 1.57 (s, 3H).

Example 16

Synthesis of 2-(4-cyanophenyl)-3-(5,6-dimethoxybenzothiophen-2-yl)-2-methylpropanoic acid N-n-butylamide: Compound (66)

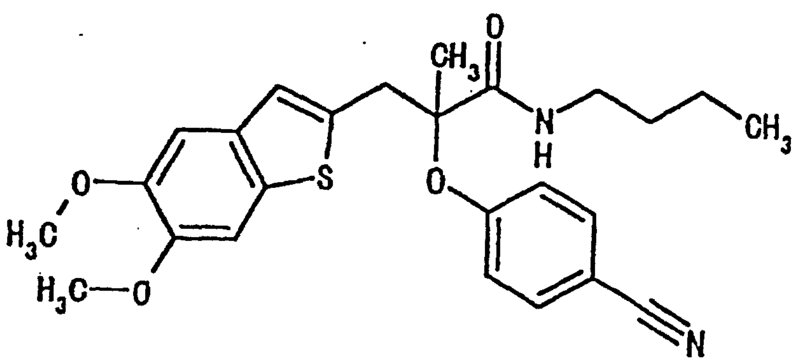

The carboxylic acid compound prepared in Example 2 (630 mg, 1.59 mmol) and n-butylamine (581 mg, 7.95 mmol) were treated as described in Example 10 to give the title compound (650 mg, 90 %) as a white amorphous solid.

$^1$H-NMR ($CDCl_3$, 270 MHz) δ = 7.60 (d, 2H, J = 9.0 Hz), 7.21 (s, 1H), 7.13 (s, 1H), 7.06 (d, 2H, J = 9.0 Hz), 6.96 (s, 1H), 6.27 (bt, 1H ,J = 5.9 Hz), 3.93 and 3.92 (2s, each 3H), 3.53 and 3.43 (2d, each 1H, J = 15.1 Hz), 3.28 - 3.11 (m, 2H), 1.58 (s, 3H), 1.30 - 1.22 (m, 2H), 1.15 - 1.05 (m, 2H), 0.76 (t, 3H, J = 7.3 Hz).

Reference Example 1

5,6-Dimethoxybenzothiophen-2-carboxyamide

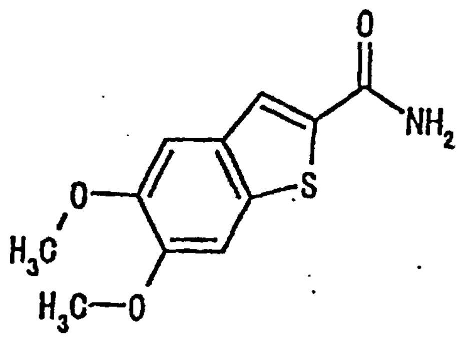

5,6-Dimethoxybenzothiophen-2-carboxylic acid (15.4 g, 64.6 mmol) was treated as described in step 3 in Example 8 to give the title compound (13.7 g, 89 %).

Melting point = 212 to 214 °C.

$^1$H-NMR (DMSO-$d_6$, 270 MHz) δ = 8.05 (bs, 1H), 7.89 (s, 1H), 7.53 (s, 1H), 7.50 (bs, 1H), 7.35 (s, 1H), 3.84 and 3.83 (2s, each 3H).

Reference Example 2

5,6-Dimethoxybenzothiophen-2-nitrile

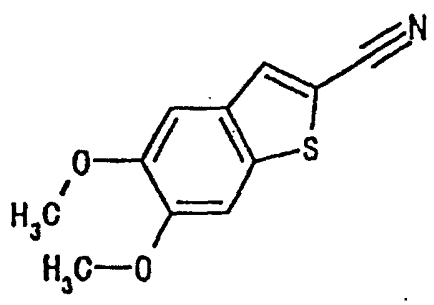

The amide compound prepared in Reference Example 1 (13.5 g, 56.9 mmol) was dissolved in pyridine (70 mL). To the solution at 10 °C or lower was added dropwise trifluoroacetic anhydride (40 mL). After stirring at 0 °C for 1 hour, water (200 mL) was added and the precipitated crystals were collected by filtration to give the title compound (11.2 g, 90 %) as opalescent crystals.

Melting point = 121 to 123 °C.

$^1$H-NMR (DMSO-$d_6$, 270 MHz) δ = 8.13 (bs, 1H), 7.66 (s, 1H), 7.48 (s, 1H), 3.88 and 3.85 (2s, each 3H).

Reference Example 3

5,6-Dimethoxybenzothiophen-2-carboxyaldehyde

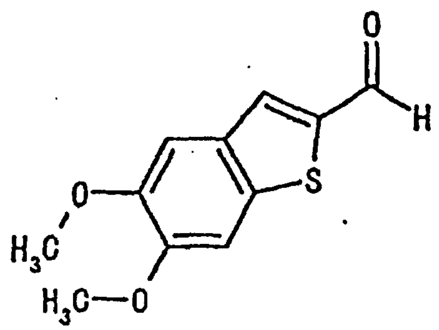

The nitrile compound prepared in Reference Example 2 (5.0 g, 22.8 mmol) was dissolved in toluene (100 mL). To the solution at -5 °C was added dropwise a 1.0 M solution of diisobutylaluminum in toluene (48 mL, 48 mmol). After stirring at room temperature for 2 hours, the mixture was poured into 1 N hydrochloric acid (500 mL) and the resulting mixture was stirred for 30 min. The mixture was extracted with dichloromethane (500 mL), dried over anhydrous magnesium sulfate and concentrated under a reduced pressure to give the title compound (4.23 g, 83 %) as opalescent crystals.

Melting point = 150 to 151 °C.

$^1$H-NMR (DMSO-$d_6$, 270 MHz) δ = 10.02 (s, 1H), 8.21 (bs, 1H), 7.62 (s, 1H), 7.55 (s, 1H), 3.89 and 3.86 (2s, each 3H).

Reference Example 4

4-Chloro-5,6-dimethoxybenzothiophen-2-carboxyaldehyde

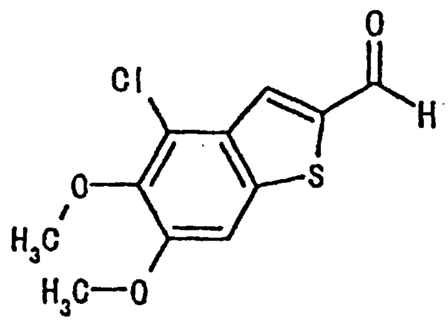

4-Chloro-5,6-dimethoxybenzothiophen-2-carboxylic acid (4.61 g, 16.9 mmol) was treated as described in Reference Examples 1 to 3 to give the title compound (2.0 g, 46 %) as opalescent crystals.

Melting point = 164 to 166 °C.

$^1$H-NMR (DMSO-$d_6$, 270 MHz) δ = 10.08 (s, 1H), 8.35 (bs, 1H), 7.81 (s, 1H), 3.95 and 3.85 (2s, each 3H).

Reference Example 5

Benzothiophen-2-methanol

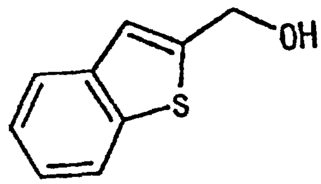

Lithium aluminum hydride (712 mg) was suspended in THF (15 mL). To the suspension at 0 °C was slowly added dropwise a solution of ethyl benzothiophen-2-carboxylate (2.06 g, 10 mmol) in THF (15 mL). After stirring at 0 °C for one hour, 1 N hydrochloric acid (150 mL) was added. After stirring for 15 min, the mixture was extracted with ethyl acetate. The organic layer was dried over anhydrous magnesium sulfate and concentrated under a reduced pressure to give the title compound (1.51 g, 92 %) as white crystals.

$^1$H-NMR ($CDCl_3$, 270 MHz) δ = 7.86 - 7.67 (m, 2H), 7.42 - 7.20 (m, 3H), 4.91 (s, 2H), 2.02 (bs, 1H).

Reference Example 6

Benzothiophen-2-methylbromide

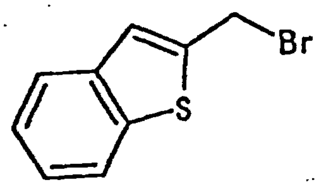

The alcohol prepared in Reference Example 5 (821 mg, 5 mmol) was dissolved in dichloromethane (5 mL). To the solution was added 47 % hydrobromic acid (5 mL) and the mixture was stirred at room temperature for 4 hours. The reaction was diluted with chloroform (50 mL), and the solution was washed with 5 % aqueous sodium bicarbonate. The organic layer was dried over anhydrous magnesium sulfate and concentrated under a reduced pressure to give the title compound (1.1 g, 97 %) as a pale yellow solid.

$^1$H-NMR ($CDCl_3$, 270 MHz) δ = 7.83 - 7.66 (m, 2H), 7.37 - 7.24 (m, 3H), 4.77 (s, 2H).

Reference Example 7

Methyl 5,6-dimethoxybenzothiophen-2-carboxylate

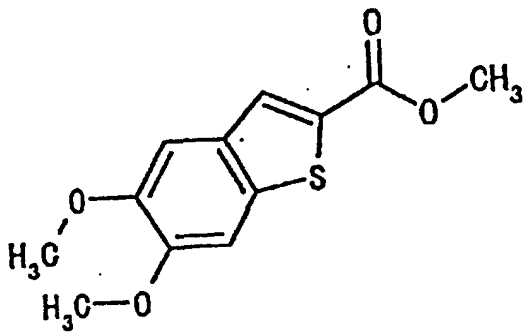

5,6-Dimethoxybenzothiophen-2-carboxylic acid (2.41 g, 10 mmol) was treated with a catalytic amount of sulfuric acid (2 mL) in methanol (110 mL) to give the title compound (2.03 g, 79 %) as white crystals.

Melting point = 154 to 156 °C.

$^1$H-NMR ($CDCl_3$, 270 MHz) $\delta$ = 7.94 (s, 1H), 7.25 (s, 1H), 7.24 (s, 1H), 3.98, 3.96 and 3.93 (3s, each 3H).

Reference Example 8

5,6-Dimethoxybenzothiophen-2-methanol

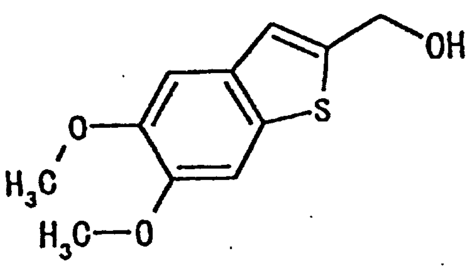

The ester compound prepared in Reference Example 7 (2.03 g, 8.0 mmol) was treated as described in Reference Example 5 to give the title compound (1.59 g, 89 %) as a pale pink amorphous solid.

$^1$H-NMR ($CDCl_3$, 270 MHz) $\delta$ = 7.25 (s, 1H), 7.15 (s, 1H), 7.09 (s, 1H), 4.88 (bs, 2H), 3.94 and 3.93 (2s, each 3H), 1.94 (bs, 1H).

Test Example 1

Evaluation of PPAR$\alpha$ and $\gamma$ agonist activity (in vitro)

It has been demonstrated that the compounds represented by formula (1) have PPAR receptor controlling activity by the following experiment.

Determination of PPARα and γ agonist activity

1) Materials for a luciferase assay using human PPARα and γ

The entire procedure was conducted using a usual yeast one- or two-hybrid system according to a basic genetic engineering process.

pGL2-UAS5-TK-luc was prepared as a reporter plasmid having a response sequence of the Ga14 protein as an yeast basic transcription factor and a luciferase gene (luc) under the control by an enhancer sequence with five times repeats of UAS and the thymidine kinase (TK) promoter promoter.

Specifically, using pRL-TK (trade name, Promega, Catalogue No. E2241) having a TK promoter as a template, a DNA fragment was amplified, which encoded the TK promoter (105/+51) downstream of an enhancer sequence in which UAS was repeated twice using 5' Primer (SEQ ID No.1):

5'-GCTAGATCT(CGACGGAGTACTGTCCTCCGAGCT)×2

CGAGGCCCCGCCCAGCGTCTTGTC-3';

and 3' Primer(SEQ ID No.2):

5'-TTAAGCTTCTGCGGCACGCTGTTGACGCTGTTAAGCGGGTCGCTGCAGGG-3'.

After cleavage with XhoI-HindIII, it was inserted into the XhoI-HindIII site upstream of the luciferase structural gene of the pGL2-Basic vector (trade name, Promega, Catalogue No. E1641) to give pGL2-UAS2-TK-luc.

Then, a synthetic DNA of an enhancer sequence after repeating the Ga14 response sequence (SEQ ID NO. 3) : 5'-ATTGGTAC(CGACGGAGTACTGTCCTCCGAGCT)×3 AGATCTCGAC was cleaved with KpnI and BglII. The DNA was inserted into the KpnI-BglII site in pGL2-UAS2-TK-luc to prepare pGL2-UAS5-TK-luc.

A vector expressing a chimera receptor in which a ligand binding region in an intranuclear receptor human PPARα or γ receptor was fused to the carbonyl terminus in the DNA binding region of the yeast Ga14 protein was prepared as follows. Specifically, using pSG5 (trade name, STRATAGENE, Catalogue No.216201) as a basic expression vector, the structural gene was replaced with that of a chimera receptor.

A DNA encoding the ligand binding region in human PPARα or γ receptor was inserted downstream of the DNA binding region in the Ga14 protein, i.e., the DNA encoding the 1st to 147th amino acid sequence such that the frames were aligned and it was inserted downstream of the promoter-enhancer region in pSG5 (trade name). For localizing the expressed chimera receptor in the nucleus, the DNA sequence was constructed such that a nuclear transition signal derived from SV-40T antigen, Ala Pro Lys Lys Lys Arg Lys Val Gly (SEQ ID NO. 4) was present at the amino terminus of the ligand binding region in the human PPARα or γ receptor.

As the structural gene segment for a ligand binding region in the human PPARα or γ receptor, a DNA was used , which encoded:

human PPARα ligand binding region: $Ser^{167}$-$Tyr^{468}$; and
human PPARγ ligand binding region: $Ser^{176}$ -$Tyr^{478}$

which correspond to $Ser^{204}$-$Tyr^{506}$ in the human PPARγ 1 receptor and PPARγ 2 receptor and thus have the same base sequence, from structural comparison for human PPAR receptors described in R. Mukherjee et al., J. Steroid Biochem. Molec. Biol., Vol. 51, p.157 (1994); M. E. Green et al. (Gene Expression., Vol.4, p.281 (1995). For monitoring an effect on basic transcription, was also prepared an expression vector having encoding only the DNA binding region of the Ga14 protein lacking the PPAR ligand binding region, the 1st to 147th amino acid sequence and a nuclear transition signal for SV-40T-antigen.

2) Luciferase assay using the human PPARα or γ receptor

CV-1 cells used as a host cell were cultured as usual. Specifically, a fetal calf serum (Intergen, Catalogue No. 1020-90) was added to the Dulbecco modified eagle medium (DMEM) to the final concentration of 10 %, and penicillin G and streptomycin sulfate were then added to the final concentrations of 50 U/mL and 50 μg/mL, respectively. In the medium, the cells were cultured at 37 °C under 5 % carbon dioxide.

The day before transfection, the cells were inoculated in a 24 well plate at 1.5 × $10^5$ cells/well in advance. LipofectAMINE (trade name, GIBCOBRL, Catalogue No.26300-61) was used for transfection. Specifically, for each well, in 40 µL of a serum-free medium Opti-MEM (trade name, GIBCOBRL, Catalogue No.31985-070) were well mixed 100 ng of a reporter plasmid, 12.5 ng of Ga14-PPAR expression vector, 200 ng of pRL-TK (trade name) as an internal control, 287.5 ng of pGEM-3Zf(+) (trade name , PROMEGA, Catalogue No. P2271) as a carrier DNA and 2.6 µL of LipofectAMINE (trade name, GIBCOBRL, Catalogue No.26300-61). To the mixture was added 170.2 µL of Opti-MEM (trade name). After washing with PBS (Phosphate Buffered Saline) and Opti-MEM (trade name), the mixture was added to the above cells. After culturing at 37 °C for 16 hours, the medium was replaced with DMEM-10 % charcoal-dextrin treated fetal calf serum (trade name, HyClone, Catalogue No. SH30068.03) containing a compound of this invention. After culturing at 37 °C for 24 hours, the cells were lysed and determined for its luciferase activity as usual.

For PPARα agonist activity, table 1 shows a relative activity for a compound of this invention at 0.1, 1.0 and 10 µM when a luciferase activity is 100 when adding Wy-14.643 (See, Cell, Vol.83, p.813 (1995), J. Biol. Chem., Vol. 270, p.12953 (1995), Proc. Natl. Acad. Sci. USA, Vol.94, p.4312 (1997), J. Biol. Chem., Vol.272, p.3406 (1997)), a positive control compound capable of significantly activating transcription of the luciferase gene to PPARα at 10 µM.

Table 1

| PPARα agonist activity | | | |
|---|---|---|---|
| Compound No. | Relative activity | | |
| | 0.1 µM | 1 µM | 10 µM |
| Example 1 (57) | 0.2 | 0.2 | 0.5 |
| Example 2 (60) | 0.1 | 0.1 | 66.4 |
| Example 3 (55) | 13.2 | 116.2 | 111.2 |
| Example 4 (82) | 0.3 | 2.9 | 248.8 |
| Example 5 (91) | 0.2 | 0.3 | 0.7 |
| Example 6 (94) | 0.1 | 0.1 | 0.1 |
| Example 7 (96) | NT | NT | NT |
| Example 8 (99) | 0.1 | 0.1 | 0.2 |
| Example 9 (101) | NT | NT | NT |
| Example 10 (105) | 0.1 | 0.1 | 20.7 |
| Example 11 (106) | 0.1 | 0.2 | 0.3 |
| Example 12 (109) | 0.2 | 0.2 | 0.2 |
| Example 13 (110) | 0.2 | 0.2 | 0.1 |
| Example 14 (119) | 0.2 | 0.2 | 0.6 |
| Example 15 (62) | 0.2 | 0.2 | 0.1 |
| Example 16 (66) | 0.2 | 0.2 | 0.1 |
| NT = Not Tested | | | |

For PPARγ agonist activity, table 2 shows a relative activity for a compound of this invention at 0.1, 1.0 and 10 µM when a luciferase activity is 100 when adding Pioglitazone (See, Cell, Vol.83, p.803 (1995), J. Biol. Chem. Vol. 270, p.12953 (1995)), a positive control compound capable of significantly activating transcription of the luciferase gene to PPARγ at 1 µM.

Table 2

| PPARγ agonist activity | | | |
|---|---|---|---|
| Compound No. | Relative activity | | |
| | 0.1 µM | 1 µM | 10 µM |
| Example 1 (57) | 0.4 | 3.2 | 81.5 |

Table 2 (continued)

| PPARγ agonist activity | | | |
|---|---|---|---|
| Compound No. | Relative activity | | |
| | 0.1 μM | 1 μM | 10 μM |
| Example 2 (60) | 17.8 | 81.9 | 78.7 |
| Example 3 (55) | 9.5 | 86.7 | 86.1 |
| Example 4 (82) | 8.0 | 68.0 | 69.9 |
| Example 5 (91) | 0.3 | 0.3 | 3.1 |
| Example 6 (94) | 1.2 | 19.0 | 60.1 |
| Example 7 (96) | NT | NT | NT |
| Example 8 (99) | 0.3 | 0.3 | 0.3 |
| Example 9 (101) | NT | NT | NT |
| Example 10 (105) | 1.2 | 2.1 | 31.2 |
| Example 11 (106) | 0.3 | 0.3 | 0.3 |
| Example 12 (109) | 0.3 | 0.3 | 0.5 |
| Example 13 (110) | 0.3 | 0.3 | 0.4 |
| Example 14 (119) | 0.3 | 0.3 | 0.5 |
| Example 15 (62) | 0.5 | 0.4 | 1.1 |
| Example 16 (66) | 0.4 | 0.4 | 0.3 |

Test Example 2

Evaluation of a relieving effect to inhibition of glucose uptake after insulin stimulation by hTNFα (in vitro)

The following experiment has demonstrated that a compound of this invention represented by formula (1) may relieve inhibition of glucose uptake after insulin stimulation by hTNFα.

The effect of relieving inhibition of glucose uptake after insulin stimulation by hTNFα in 3T3-L1 adipose cells was studied by determining a glucose level in a medium.

Specifically, murine 3T3-L1 fibroblasts (Dainippon Pharmaceutical Co., Ltd.) was suspended in a Dulbecco modified eagle medium (DMEM) containing 10 % bovine serum. The suspension was inoculated to a 24 well collagen-coated plate and cultured to be confluent. Then, it was further cultured for 2 days (the day when this culturing ended was defined as differentiation induction day 0). The medium was then replaced with a differentiation induction medium (DMEM containing 10 % fetal calf serum, 0.5mM 3-isobutyl-1-methyl-xanthine, 0.25 μM dexamethasone and 1 μg/mL insulin), and then it was cultured for 40 hours. On differentiation induction day 2, the medium was replaced with DMEM containing 10 % fetal calf serum and 1 μg/mL insulin; and on differentiation induction day 4, the medium was replaced with DMEM containing 10 % fetal calf serum and 50 ng/mL insulin. Culturing was continued. On differentiation induction day 7 when the cells have been fully differentiated into adipose cells, a compound of this invention was added to the DMEM containing 10 % fetal calf serum, 50 ng/mL insulin and 5 ng/mL hTNFα, and culturing was continued. The compound of this invention was dissolved in DMSO and then added to the medium to the final DMSO concentration of 0.1 %. On differentiation induction day 9, the medium was replaced with a medium containing the compound of this invention and having the same composition as that on differentiation induction day 7. In the above culturing, all the mediums contained 50 U/mL penicillin G and 50 μg/mL streptomycin sulfate for preventing contamination and culturing was conducted at 37 °C under 5 % carbon dioxide.

For eliminating effects of the serum, the medium was replaced with a DMEM containing 2 % bovine serum albumin (BSA) on differentiation induction day 11, and then it was cultured for 4 hours. After removing the medium, the cells were washed with Krebs-Ringer buffer (1.2 mM $KH_2PO_4$, 4.7mM KCl, 118mM NaCl, 25mM $NaHCO_3$, 2.5mM $CaCl_2$, pH 7.4) containing 0.1 % BSA and 180 mg/L glucose. After adding Krebs-Ringer buffer containing 0.1 % BSA, 180mg/L glucose and 0.5 ng/mL insulin at 300 μL per well, the cells were cultured at 37 °C under 5 % carbon dioxide for 3 hours.

A glucose level as an indicator of glucose uptake in a culture supernatant was determined using Glucose CII Test Wako (Wako Pure Chemical Industries).

Activities of the compounds according to this invention (10 μM) (relieving effect of glucose uptake inhibition induced by hTNFα) are represented as a relative value when the activity of the positive control compound (Pioglitazone 10 μM) is 100. The results are shown in Table 3.

Table 3

| Release of inhibition of glucose metabolism by hTNFα using a compound | |
|---|---|
| Compound No. | Relative Activity |
| Example 1 | NT |
| Example 2 | 103 |
| Example 3 | 87 |
| Example 4 | 132 |
| Example 5 | 10 |
| Example 6 | 84 |
| Example 7 | 20 |
| Example 8 | 0 |
| Example 9 | 30 |
| Example 10 | 115 |
| Example 11 | 5 |
| Example 12 | 0 |
| Example 13 | 5 |
| Example 14 | 18 |
| Example 15 | NT |
| Example 16 | NT |
| NT = Not Tested | |

Test Example 3

Evaluation of hypoglycemic and hypolipidemic effects using a diabetic model mouse (KKAy mouse) ( in vivo)

Type II diabetic mice "KK-Ay/Ta Jci" (male, Nippon Clare, 10 weeks, five per a group) in each cage were used. In the morning of the initial day of the study, blood was collected from an orbital vein. A glucose level was determined using a new blood sugar test (Boehringer Mannheim) for a centrifuge supernatant after deproteination of the blood with perchloric acid. The blood was centrifuged to prepare a plasma. Then, a triglyceride and a free fatty acid levels in the plasma were determined using Triglyceride E-Test Wako and NEFA-C Test Wako (Wako Pure Chemicals, Inc.), respectively. After dividing the animals into groups such that each group had an equal blood glucose level, the compound in Example 2 was suspended in 0.5 % aqueous CMC solution and the suspension was orally administered once daily for four days. On the fifth day of the study, a blood was collected for determination of a blood glucose level, a triglyceride level and a free fatty acid level. A group to which only 0.5 % aqueous CMC solution was administered was used as a control group while a group to which pioglytazone was administered was used as a positive control. A reduction rate for a parameter in each group was calculated using the following equation. The results are shown in Table 4.

Table 4

| Evaluation results of in vivo hypoglycemic and hypolipidemic effects using KKAy mice | | | | |
|---|---|---|---|---|
| Compound group | Dose (mg/kg) | Reduction rate of a blood glucose level (%) | Reduction rate of a triglyceride level (%) | Reduction rate of a free fatty acid level (%) |
| Control | - | 7 | -11 | -7 |
| Example 4 | 30 | 58 | 85 | 69 |
| Pioglytazone | 30 | 28 | 60 | 49 |

Reduction rate (%) = {1 - (a parameter value on the fifth day in each group) / (a parameter value on the first day in each group)} $\times$ 100

Industrial Applicability

The compounds of this invention are novel substances and potently agonize PPARα or γ which is an intranuclear transcription factor, as demonstrated in Examples and Test Examples. The compounds are less toxic so that they may be promising as a drug for preventing or treating a variety of diseases involving PPARα or γ.

## Claims

**1.** A benzothiophene derivative represented by formula (1) or a pharmaceutically acceptable salt thereof.

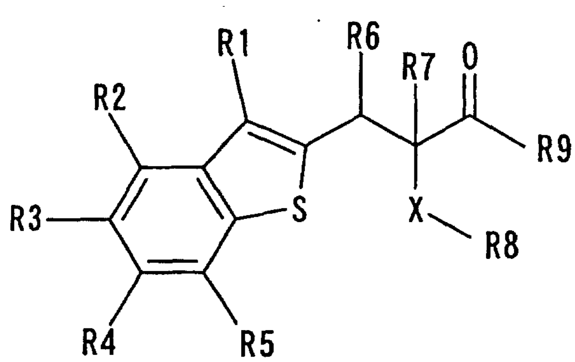

(1)

wherein $R^1$, $R^2$, $R^3$, $R^4$ and $R^5$ independently represent hydrogen, halogen, hydroxy, lower alkyl having 1 to 4 carbon atoms, lower alkyloxy having 1 to 4 carbon atoms, nitro, carboxyl, optionally substituted carbamoyl, lower alkoxycarbonyl having 1 to 4 carbon atoms, optionally substituted phenoxy, thiol, lower alkylthiol having 1 to 4 carbon atoms, optionally substituted phenylthio, amino optionally substituted with lower alkyl having 1 to 4 carbon atoms, amino optionally substituted with lower alkylcarbonyl having 1 to 4 carbon atoms or amino substituted with optionally substituted benzoyl; $R^6$ represents hydrogen or lower alkyl having 1 to 4 carbon atoms; $R^7$ represents hydrogen, lower alkyl having 1 to 4 carbon atoms or lower alkyloxy having 1 to 4 carbon atoms; or $R^6$ and $R^7$ may be directly bound together to form a carbon-carbon double bond; $R^8$ represents hydrogen, lower alkyl having 1 to 4 carbon atoms, optionally substituted phenyl, lower alkyloxy having 1 to 4 carbon atoms, amino optionally substituted with lower alkyl having 1 to 4 carbon atoms or optionally substituted phenylamino; $R^9$ represents hydroxy, lower alkyloxy having 1 to 4 carbon atoms, optionally substituted phenyloxy, amino optionally substituted with lower alkyl having 1 to 4 carbon atoms or optionally substituted phenylamino; and X represents oxygen, sulfur or carbonyl.

**2.** A benzothiophene derivative represented by formula (2) or a pharmaceutically acceptable salt thereof.

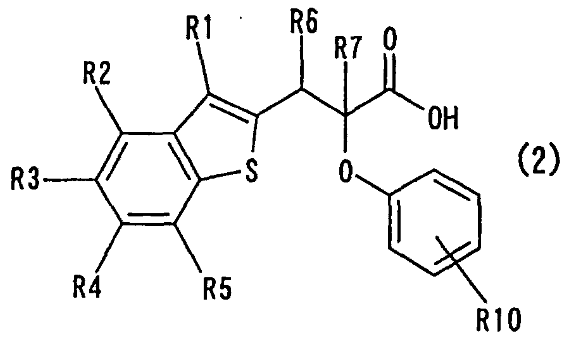

(2)

wherein $R^1$, $R^2$, $R^3$, $R^4$, $R^5$, $R^6$ and $R^7$ are as defined in formula (1); $R^{10}$ represents hydrogen, hydroxy, lower alkyl having 1 to 4 carbon atoms, lower alkoxy having 1 to 4 carbon atoms, nitro, halogen, carboxyl, lower alkoxycarbonyl having 1 to 4 carbon atoms, optionally substituted phenyl, optionally substituted amino, optionally substituted carbamoyl or optionally substituted amidino.

**3.** A benzothiophene derivative represented by formula (3) or a pharmaceutically acceptable salt thereof.

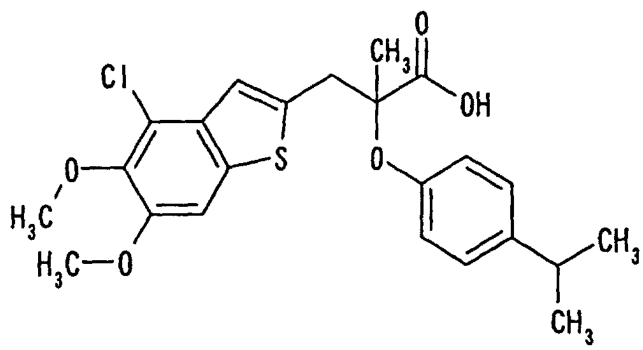

(3)

**4.** An agonist for a peroxisome proliferator-activated receptor (PPAR) α or γ which is an intranuclear transcription factor comprising the benzothiophene derivative as claimed in Claim 1, 2 or 3 as an active ingredient.

**5.** A drug for preventing or treating diabetes comprising the benzothiophene derivative as claimed in Claim 1, 2 or 3 as an active ingredient.

**6.** A drug for preventing or treating hyperlipidemia comprising the benzothiophene derivative as claimed in Claim 1, 2 or 3 as an active ingredient.

**7.** A drug for preventing or treating arteriosclerosis comprising the benzothiophene derivative as claimed in Claim 1, 2 or 3 as an active ingredient.

**INTERNATIONAL SEARCH REPORT**

International application No.
PCT/JP01/02170

A. CLASSIFICATION OF SUBJECT MATTER
Int.Cl[7] C07D333/60, A61K31/381, 5377, A61P43/00, 3/06, 9/10

According to International Patent Classification (IPC) or to both national classification and IPC

B. FIELDS SEARCHED
Minimum documentation searched (classification system followed by classification symbols)
Int.Cl[7] C07D333/60, A61K31/381, 5377, A61P43/00, 3/06, 9/10

Documentation searched other than minimum documentation to the extent that such documents are included in the fields searched

Electronic data base consulted during the international search (name of data base and, where practicable, search terms used)
CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | Chemical Abstracts, vol.71, abstract no.31697 (RN=30126-02-4) & J. Chem. Soc., C, (1970), (18), pp.2431-5 | 1 |
| A | WO, 97/31907, A1 (GLAXO GROUP LTD.), 04 September, 1997 (04.09.97), & AU, 9720935, A & ZA, 9701645, A & NO, 9803940, A & EP, 888317, A1 & CZ, 9802750, A & SK, 9801163, A & CN, 1218460, A & BR, 9707786, A & JP, 2000-507216, A & NZ, 331381, A & KR, 99087321, A & TW, 391958, A | 1-7 |
| A | EP, 540051, A1 (DAIICHI PHARM. CO., LTD.), 05 May, 1993 (05.05.93), & AU, 9227470, A & NO, 9204164, A & CA, 2081836, A & FI, 9204932, A & CZ, 9203276, A & ZA, 9208276, A & JP, 5-208946, A & TW, 210998, A & CN, 1072677, A & NZ, 244936, A & US, 5576343, A & US, 5620991, A & JP, 10-291931, A & US, 5866577, A | 1-7 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 May, 2001 (08.05.01) | 22 May, 2001 (22.05.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP01/02170

C (Continuation). DOCUMENTS CONSIDERED TO BE RELEVANT

| Category* | Citation of document, with indication, where appropriate, of the relevant passages | Relevant to claim No. |
|---|---|---|
| | & IL, 103564, A & SK, 9203276, A<br>& US, 5962685, A & SG, 78251, A | |

Form PCT/ISA/210 (continuation of second sheet) (July 1992)